# FASCICULE DE BREVET EUROPEEN

(11) **EP 1 018 333 B1**
(45) Date de publication et mention de la délivrance du brevet: **01.03.2006**
(21) Numéro de dépôt: 99403170.6
(22) Date de dépôt: 16.12.1999
(51) Int. Cl.: A61Q 5/06, A61K 8/41

(54) **Utilisation de nitroanilines cationiques monobenzéniques en teinture des fibres kérantiques, compositions tinctoriales at procédés de teinture**
Verwendung von kationischen Monobenzolanilinen zur Färbung von Keratinfasern, Zusammensetzungen und Färbeverfahren
Use of cationic monobenzene nitroanilines for dyeing keratin fibres , compositions and dyeing process

(30) Priorité: 08.01.1999 FR 9900151
(43) Date de publication de la demande: 12.07.2000
(73) Titulaire: L'OREAL, 75008 Paris (FR)
(72) Inventeur: Genet, Alain, 93600 Aulnay sous Bois (FR); Lagrange, Alain, 77700 Coupvray (FR)
(74) Mandataire: Miszputen, Laurent

(56) Documents cités:
- EP-A- 0 314 162
- WO-A-99/03836
- BE-A- 616 439
- DE-A- 4 404 198
- DE-A- 19 802 940
- FR-A- 1 221 122
- FR-A- 1 565 247
- GB-A- 1 531 605
- US-A- 5 169 403
- US-A- 5 256 823
- CHEMICAL ABSTRACTS, vol. 72, no. 2, 12 janvier 1970 (1970-01-12) Columbus, Ohio, US; abstract no. 4329y, M. IIZUKA ET AL: "Cationic azo dyes" page 52; XP002115606 & JP 06 910910 A (HODOGAYA) 20 mai 1969 (1969-05-20)

## Description

La présente invention a pour objet l'utilisation de nitroanilines monobenzéniques comportant au moins un groupement cationique choisi parmi des chaînes aliphatiques comportant au moins une charge cationique délocalisée sur un hétérocycle polyazoté insaturé à 5 chaînons, leur utilisation à titre de colorant direct dans les applications de teinture des matières kératiniques, en particulier des fibres kératiniques humaines, et notamment les cheveux, et plus particulièrement les compositions de teinture les renfermant.

Il est connu de teindre les fibres kératiniques, et en particulier les cheveux, avec des compositions tinctoriales contenant des colorants directs, c'est-à-dire des molécules colorantes ayant une affinité pour lesdites fibres. Le procédé de teinture qui les met en oeuvre est un procédé dit de coloration directe qui consiste à laisser pauser les colorants directs sur les fibres, puis à les rincer.
Les colorations qui en résultent sont des colorations temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Parmi les colorants directs connus, on a certes déjà décrit des nitroanilines cationiques mais leur charge cationique est localisée sur l'atome d'azote d'une chaîne aliphatique. De telles nitroanilines sont par exemple décrites dans les brevets américains N°- 5 135 543, 5 256 823 et 5 169 403 ;dans les demandes de brevet EP 314162, DE 4404198.

On connaît aussi l'utilisation en tant que colorant direct, des composés de la famille des composés nitrés aromatiques porteurs d'une fonction éther (GB1531605) dont certains peuvent être cationiques. Cependant, ils ne portent pas de charge cationique délocalisée sur un hétérocycle polyazoté insaturé.
Enfin la demande de brevet BE 616439 décrit des colorants directs portant une charge cationique délocalisée sur un hétérocycle azoté, mais ces colorants dérivent des colorants azoïques, anthraquinoniques et phtalocyaniniques.
Cependant, en teinture capillaire, on recherche en permanence des colorants directs qui présentent des caractéristiques toujours plus performantes.

C'est donc après d'importantes recherches menées sur la question que la demanderesse vient maintenant de découvrir de façon totalement inattendue et surprenante, que des nitroanilines cationiques monobenzéniques, dont au moins surprenante, que des nitroanilines cationiques monobenzéniques, dont au moins une charge cationique est délocalisée sur un hétérocycle polyazoté insaturé à 5 chaînons et comportant donc au moins un groupement cationique Z, Z étant choisi parmi des chaînes aliphatiques quaternisées, des chaînes aliphatiques comportant au moins un cycle saturé quaternisé et des chaînes aliphatiques comportant au moins un cycle insaturé quaternisé, conviennent pour une utilisation comme colorants directs pour la teinture directe, mais en outre qu'ils permettent d'obtenir des compositions tinctoriales conduisant à des colorations puissantes et variées présentant d'excellentes propriétés de résistance aux diverses agressions que peuvent subir les cheveux ( lumière, frottement, intempéries, frottement, shampooings, transpiration) et notablement améliorées, par rapport à celles des teintures réalisées avec des nitroanilines cationiques connues de l'art antérieur dont la charge cationique est localisée sur l'atome d'azote d'une chaîne aliphatique.

Enfin, ces nitroanilines présentent une meilleure solubilité dans les milieux classiquement utilisés pour la teinture des fibres kératiniques et s'avèrent aisément synthétisables.

Ces découvertes sont à la base de la présente invention.

La présente invention a ainsi pour objet, l'utilisation, à titre de colorants directs dans des, ou pour la fabrication de, compositions tinctoriales pour matières kératiniques, et notamment pour fibres kératiniques humaines tells que les cheveux, de nitroanilines cationiques monobenzéniques de formule (I) suivante, et leurs sels d'addition avec un acide : 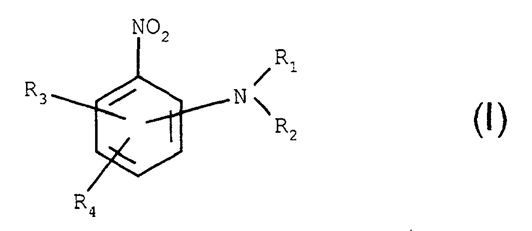 formule dans laquelle,
- **R**_{**1**}**, R**_{**2**}**,** qui peuvent être identiques ou différents, représentent un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆; un radical aryle ; un radical benzyle ; un radical cyanoalkyle en C₁-C₆; un radical carbamylalkyle en C₁-C₆ ; un radical N-alkyl(C₁-C₆)carbamylalkyle en C₁-C₆ ; un radical N,N-dialkyl(C₁₋C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁₋C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁₋C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁₋C₆)carbonyle, carbamyle, N-alkyl(C₁-C₆)carbamyle ou N,N-dialkyl(C₁₋C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z défini ci-après ; ou pouvant former ensemble, avec l'atome d'azote auquel ils sont rattachés, un cycle à 5 ou 6 chaînons, carboné, ou contenant un ou plusieurs hététoatomes ;
- **R**_{**3**}**, R**_{**4**}**,** qui peuvent être identiques ou différents, représentent un atome d'hydrogène ; un atome d'halogène ; un groupement Z défini ci-après ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁₋C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁₋C₆)carbonylalkyle(C₁-C₆) ; un radical N-alkyl(C₁-C₆)aminoalkyl(C₁₋C₆)carbonylalkyle(C₁-C₆) ; un radical N,N-dialkyl(C₁-C₆)aminoalkyl(C₁₋C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁₋C₆)aminosulfonylalkyle(C₁-C₆) ; un radical N,N-dialkyl(C₁₋C₆)aminosulfonylalkyle(C₁-C₆) ; un radical carbamyle ; un radical N-alkyl(C₁₋C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un radical aminoalkyle(C₁-C₆) dont l'alkyle est substitué ou non substitué par un ou plusieurs radicaux hydroxy et dont l'amine est substituée ou non substituée par un ou deux radicaux, identiques ou différents, choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁₋C₆)carbonyle, alkyl(C₁-C₆)carboxy, thiocarbamyle, ou par un groupement Z défini ci-après ; un groupement OR₅ ou -SR₅ défini ci-après ; ou pouvant former ensemble, avec l'atome d'azote auquel ils sont rattachés, un cycle à 5 ou 6 chaînons, carboné, ou contenant un ou plusieurs hététoatomes ;
- **R**_{**5**} désigne un atome d'hydrogène ; un radical alkyle en C₁-C₆; un radical monohydroxyalkyle en C₁-C₆ ; un radical polyhydroxyalkyle en C₂-C₆ ; un groupement Z ; un radical alcoxy(C₁-C₆)alkyle en C₁-C₆ un radical aryle ; un radical benzyle ; un radical carboxyalkyle en C₁-C₆ ; un radical alkyl(C₁₋C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux, identiques ou différents, choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁₋C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou par un groupement Z défini ci-après ; ou pouvant former ensemble, avec l'atome d'azote auquel ils sont rattachés, un cycle à 5 ou 6 chaînons, carboné, ou contenant un ou plusieurs hététoatomes ;
- **Z** est choisi parmi les groupements cationiques insaturés de formules (II) et (III) suivantes, et les groupements cationiques saturés de formule (IV) suivante : 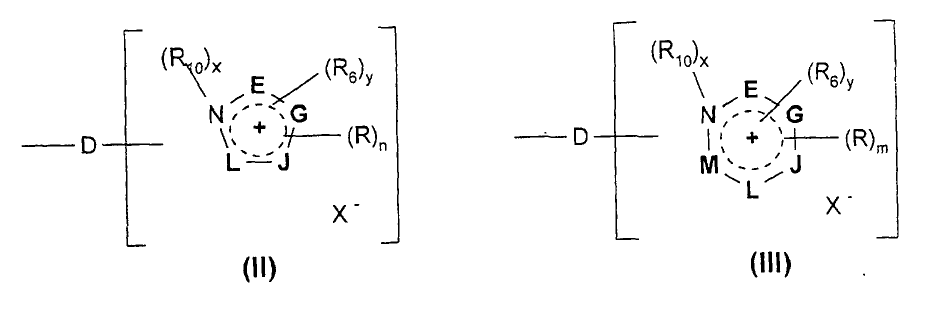 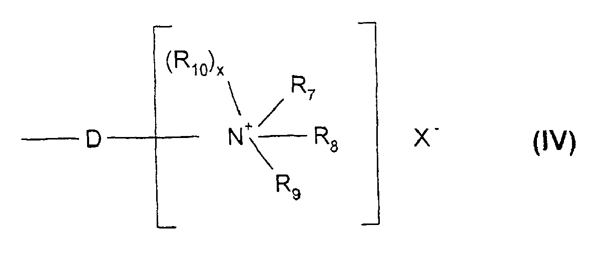
   dans lesquelles :
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement;
   - les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁₋C₆)thio, un radical amino, un radical amino protégé par un radical alkyl(C₁₋C₆)carbonyle, carbamyle ou alkyl(C₁-C₆)suifonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
   - R₆ représente un radical alkyle en C₁-C₆, un radical monohydroxyalkyle en C₁-C₆, un radical polyhydroxyalkyle en C₂-C₆, un radical cyanoalkyle en C₁₋C₆, un radical trialkyl(C₁-C₆)silanealkyle en C₁-C₆, un radical alcoxy(C₁₋C₆)alkyle en C₁-C₆, un radical carbamylalkyle C₁-C₆, un radical alkyl(C₁₋C₆)carboxyalkyle en C₁-C₆, un radical benzyle, un second groupement Z identique ou différent du premier groupement Z ;
   - R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialky/(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₇, R₈ et R₉ peuvent également former ensemble, avec l'atome d'azote auquel ils sont rattachés, un cycle saturé à 5 ou 6 chaînons, carboné, ou pouvant contenir un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁₋C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
      l'un des radicaux R₇, R₈ et R₉ peut également représenter un second groupement Z, identique ou différent du premier groupement Z ;
   - R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl (C₁-C₆) carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁₋C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁₋C₆)sulfonamidoalkyle en C₁-C₆ ;
   - x et y sont des nombres entiers égaux à 0 ou 1 ; avec les conditions suivantes :
      - dans les groupements cationiques insaturés de formule (II) :
         - lorsque x = 0, le bras de liaison D est rattaché à l'atome d'azote,
         - lorsque x = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₆ est porté par l'atome d'azote du cycle insaturé ; ou bien
            2) lorsqu'au moins un des sommets E, G, J et L représente un atome d'azote sur lequel le radical R₆ est fixé ;
      - dans les groupements cationiques insaturés de formule (III) :
         - lorsque x = 0, le bras de liaison D est rattaché à l'atome d'azote,
         - lorsque x = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₆ est porté par l'atome d'azote du cycle insaturé ;
      - dans les groupements cationiques de formule (IV) :

      - lorsque x = 0, alors le bras de liaison D est rattaché à l'atome d'azote portant les radicaux R₇ à R₉,
      - lorsque x = 1, alors deux des radicaux R₇ à R₉ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
   - **X**⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;

**étant entendu :**
- que le nombre de groupements cationiques insaturés Z de formule (II), dans lesquels au moins un des sommets E, G, J et L représente un atome d'azote, est au moins égal à 1, et
- que, lorsque un et un seul des radicaux R₁ ou R₂ ou R₅ désigne un groupement Z dans lequel le bras de liaison D représente une chaîne alkyle comportant une fonction cétone, alors ladite fonction cétone n'est pas directement rattachée à l'atome d'azote du groupement NR₁R₂ ou à l'atome d'oxygène du groupement OR₅.

Les radicaux alkyles et alcoxy cités ci-avant dans les formules (I), (II), (III) et (IV) peuvent être linéaires ou ramifiés.

Les nitroanilines cationiques monobenzéniques de formule (I) peuvent être éventuellement salifiées par des acides minéraux forts tels que HCl, HBr, H₂SO₄, ou des acides organiques tels qu'acétique, lactique, tartrique, citrique ou succinique.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les nitroanilines cationiques monobenzéniques de formule (I) ci-dessus, on peut notamment citer les composés suivants :
- le méthylsulfate du 3-[3-(4,5-dichloro-2-nitro-phénylamino)-propyl]-1-méthyl-3H-imidazol-1-ium,
- le méthylsulfate du 3-[3-(4-chloro-5-méthoxy-2-nitro-phénylamino)-propyl]-1-méthyl-3H-imidazol-1-ium,
- le méthylsulfate du 3-[3-(4-chloro-5-méthylsulfanyl-2-nitro-phénylamino)-propyl]-1-méthyl-3H-imidazol-1-ium,
- le méthylsulfate du 1,3-diméthyl-2-(3-méthylamino-4-nitro-phénylsulfanyl)-3H-imidazol-1-ium,
- le bromure du 1-{2-[5-(2-hydroxy-éthylamino)-2-méthyl-4-nitro-phénoxy]-éthyl}-3-méthyl-3-H-imidazol-1-ium,
- le méthylsulfate, hydrate, du 3-[3-(4-méthoxy-2-nitro-phénylamino)-propyl]-1-méthyl-3H-imidazol-1-ium,
- le méthylsulfate du 3-{3-[4-(2-hydroxyéthoxy)-2-nitro-phénylamino]-propyl}-1-méthyl-3H-imidazol-1-ium,
- le bromure du 3-méthyl-1-[2-(4-nitro-phénylamino)-éthyl]-3-H- imidazol-1-ium,
- le méthylsulfate du 1-méthyl-3-[3-(4-nitro-phénylamino)-propyl]-3-H-imidazol-1-ium,
- le chlorhydrate, chlorure du 1-[3-(2-amino-5-nitro-phénoxy)-propyl]-3méthyl-3H-imidazol-1-ium,
- le chlorure, hydrate de 3-[3-(4,5-dichloro-2-nitro-phénylamino)-propyl]-1-(3-triméthylsilanyl-propyl]-3-H-imidazol-1-ium,
- le bromure de 1-[3-(2-amino-5-nitro-phénoxy)-propyl]-2-méthyl-2H-pyrazol-1-ium,
- le méthylsulfate de 1-méthyl-3-[3-(2-nitro-phénylamino)-propyl]-3-H-imidazol-1-ium,
- le chlorure de 1-(3-chloro-propyl)-3-[3-(4-nitro-phénylamino)-propyl]-3-H-imidazol-1-ium,
- le chlorure de 1-(2-hydroxy-éthyl)-3-[3-(4-nitro-phénylamino)- propyl]-3-H-imidazol-1-ium,
- le méthylsulfate de 3-[3-(4-benzyloxy-2-nitro-phénylamino)- propyl]-1-méthyl-3-H-imidazol-1-ium,
- le méthylsulfate de 3-[3-(2-cyano-4-nitro-phénylamino)- propyl]-1-méthyl-3-H-imidazol-1-ium,
- le méthylsulfate de 1-méthyl-3-[3-(2-méthyl-4-nitro-phénylamino)- propyl]-3-H-imidazol-1-ium,
- le méthylsulfate de 3-[3-(2-fluoro-4-nitro-phénylamino)- propyl]-1-méthyl-3-H-imidazol-1-ium,
- le bromure de 1-[2-(2-méthoxy-4-nitro-phénylamino)-éthyl]-3-méthyl-3-H-imidazol-1-ium,
- le méthylsulfate de 3-[3-(3-hydroxy-4-nitro-phénylamino)- propyl]-1-méthyl-3-H-imidazol-1-ium,
- le méthylsulfate de 3-[3-(2-chloro-4-nitro-phénylamino)- propyl]-1-méthyl-3-H-imidazol-1-ium,
- le bromure de 2-méthyl-1-[2-(4-nitro-phénylamino)-éthyl]-2-H-pyrazol-1-ium,
- le bromure de 1-(3-bromo-2-hydroxy-propyl)-3-[3-(4-nitro-phénylamino)-propyl]-3-H-imidazol-1-ium,
- le dichlorure de 1-(3-triméthylammonium-2-hydroxy-propyl)-3-[3-(4-nitro-phénylamino)-propyl]-3H-imidazol-1-ium,
- le chlorure de diéthyl-(2-hydroxy-éthyl)-[4-(4-nitro-phénylamino)-pentyl]-ammonium,
- le méthylsulfate de 3-[3-(4-chloro-2-nitro-phénylamino)-propyl]-1-méthyl-3H-imidazol-1-ium,
- le méthylsulfate de 3-[3-(2-chloro-6-nitro-phénylamino)-propyl]-1-méthyl-3H-imidazol-1-ium,
- le chlorure de 1-méthyl-3-[2-(4-nitro-phénylamino)-butyl]-3H-imidazol-1-ium,
- le chlorure de 3-méthyl-1-[2-méthyl-2-(4-nitro-phénylamino)-propyl]-3H-imidazol-1-ium,
- le chlorure de 1-{2-[(2-(3-méthyl-3H-imidazol-1-ium)-éthyl)-(4-nitro-phényl)-amino]-éthyl}-3-méthyl-3H-imidazol-1-ium,

et plus préférentiellement parmi eux, on choisit les composés de formules (I)₁ à (I)₁₁ suivantes : 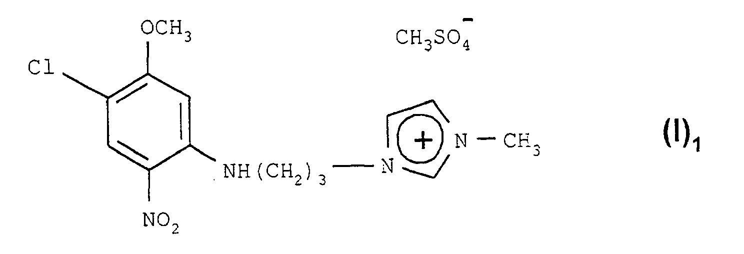
i.e. méthylsulfate du 3-[3-(4-chloro-5-méthoxy-2-nitro-phénylamino)-propyl]-1-méthyl-3H-imidazol-1-ium, 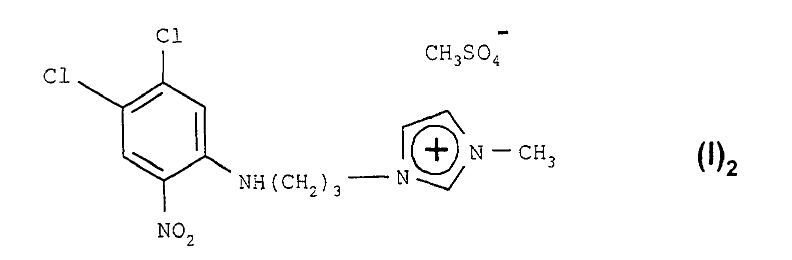
i.e. méthylsulfate du 3-[3-(4,5-dichloro-2-nitro-phénylamino)-propyl]-1-méthyl-3H-imidazol-1-ium, 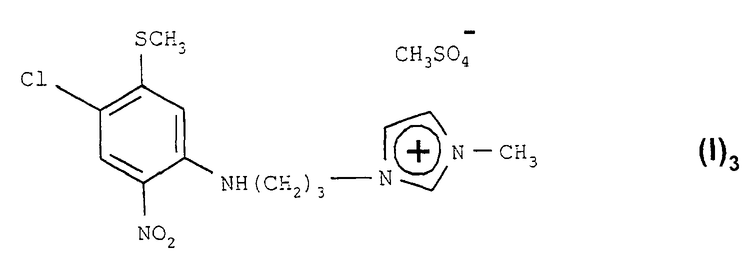
i.e. méthylsulfate du 3-[3-(4-chloro-5-méthylsulfanyl-2-nitro-phénylamino)-propyl]-1-méthyl-3H-imidazol-1-ium, 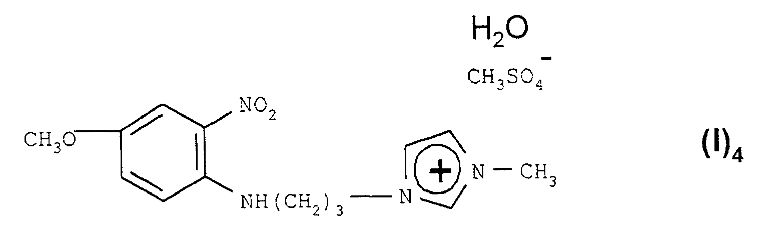
i.e. méthylsulfate, hydrate, du 3-[3-(4-méthoxy-2-nitro-phénylamino)-propyl]-1- méthyl-3H-imidazol-1-ium, 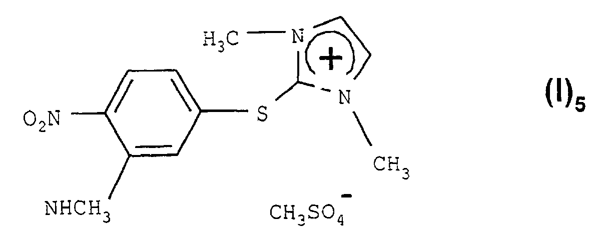
i.e. méthylsulfate du 1,3-diméthyl-2-(3-méthylamino-4-nitro-phénylsulfanyl)-3H-imidazol-1-ium, 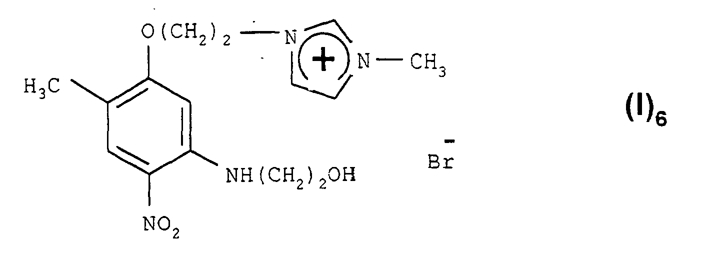
i.e. bromure du 1-{2-[5-(2-hydroxy-éthylamino)-2-méthyl-4-nitro-phénoxy]-éthyl}-3-méthyl-3-H-imidazol-1-ium, 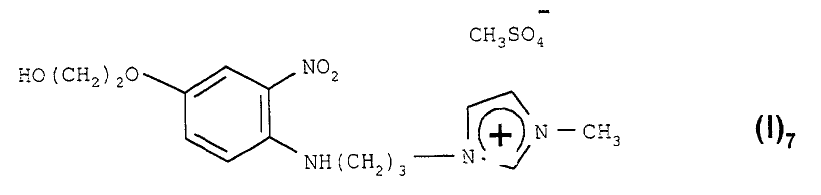
i.e. méthylsulfate du 3-{3-[4-(2-hydroxyéthoxy)-2-nitro-phénylamino]-propyl}-1-méthyl-3H-imidazol-1-ium, 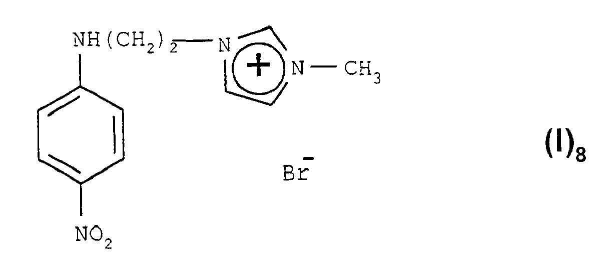
i.e. bromure du 3-méthyl-1-[2-(4-nitro-phénylamino)-éthyl]-3-H-imidazol-1-ium, 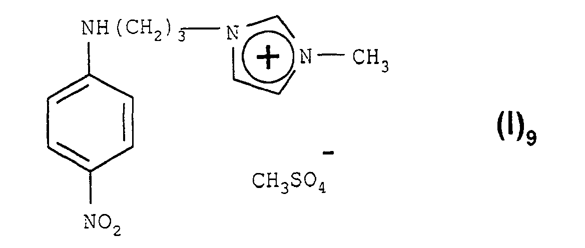
i.e. méthylsulfate du 1-méthyl-3-[3-(4-nitro-phénylamino)-propyl]-3-H-imidazol-1-ium, 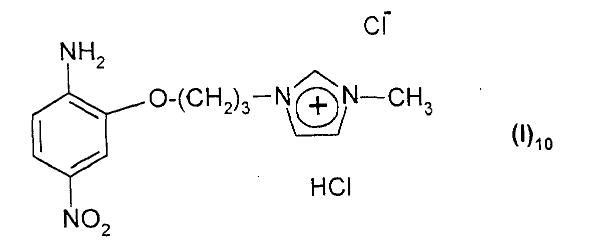
i.e. Chlorhydrate, chlorure du 1-[3-(2-amino-5-nitro-phénoxy)-propyl]-3méthyl-3H-imidazol-1-ium 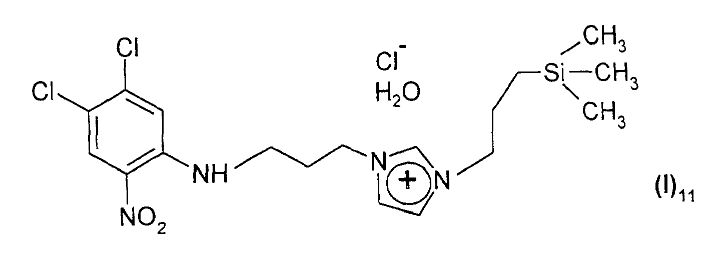
i.e.chlorure, hydrate de 3-[3-(4,5-dichloro-2-nitro-phénylamino)-propyl]-1-(3-triméthylsilanyl-propyl]-3-H-imidazol-1-ium.

Les nitroanilines cationiques monobenzéniques de formule (I) conformes à l'invention peuvent être facilement obtenues selon des méthodes généralement bien connues de l'état de la technique pour l'obtention des amines quaternisées, par exemple :
- en un temps, par condensation d'une nitroaniline comportant un radical halogénoalkyle avec un composé porteur d'un radical amine tertiaire, ou par condensation d'une nitroaniline comportant un radical amine tertiaire avec un composé porteur d'un radical halogénoalkyle ;
- ou en deux temps, par condensation d'une nitroaniline comportant un radical halogénoalkyle avec un composé porteur d'une amine secondaire, ou par condensation d'un ortho ou para halogéno-nitrobenzène avec une amino(di-substituée)alkyleamine, suivie d'une quaternisation avec un agent alkylant.

L'étape de quaternisation est, généralement par commodité, la dernière étape de la synthèse, mais peut intervenir plus tôt dans la suite des réactions conduisant à la préparation des composés de formule (I).

L'invention a aussi pour objet une composition de teinture des matières kératiniques, caractérisée par le fait qu'elle comprend, dans un milieu approprié pour la teinture, une quantité efficace d'au moins une nitroaniline cationique monobenzénique de formule (I) décrite ci-avant.

L'invention a également pour objet une composition pour la teinture directe des fibres kératiniques humaines, telles que les cheveux, caractérisée par le fait qu'elle comprend, dans un milieu approprié pour la teinture, une quantité efficace d'au moins une nitroaniline cationique monobenzénique telle que définie ci-avant par la formule (I).

L'invention a pour autre objet l'utilisation des nitroanilines cationiques monobenzéniques de formule (I), à titre de colorants directs, dans des, ou pour la préparation de, compositions tinctoriales pour matières kératiniques.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Le ou les nitroaniline(s) cationique(s) monobenzénique(s) de formule (I) conformes à l'invention et/ou le ou leurs sels d'addition avec un acide représentent de préférence de 0,005 à 12% en poids environ du poids total de la composition tinctoriale, et encore plus préférentiellement de 0,05 à 6% en poids environ de ce poids.

Les nitroanilines cationiques monobenzéniques de formule (I) peuvent également servir, dans les procédés bien connus de teinture d'oxydation, utilisant des colorants d'oxydation (précurseurs de colorants d'oxydation et éventuellement des coupleurs), à nuancer ou enrichir de reflets les teintures obtenues avec les colorants d'oxydation.

La composition tinctoriale selon l'invention peut encore contenir, pour élargir la palette de nuances et obtenir des teintes variées, outre les nitroanilines cationiques monobenzéniques de formule (I), d'autre(s) colorant(s) direct(s) classiquement utilisés, et notamment, des colorants nitrés benzéniques autres que les nitroanilines cationiques monobenzéniques de formule (I) selon la présente invention et tels que les nitrophénylènediamines, les nitrodiphénylamines, les éthers de phénol nitrés ou les nitrophénols, des nitropyridines, des colorants anthraquinoniques, des colorants mono- ou diazoïques, triarylméthaniques, aziniques, acridiniques et xanthéniques, ou encore des colorants métallifères.
La proportion de tous ces autres colorants directs d'addition peut varier entre environ 0,05 et 10% en poids par rapport au poids total de la composition tinctoriale.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁₋C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.
Les solvants peuvent être présents dans des proportions de préférence comprises entre 1 et 40% en poids par rapport au poids total de la composition tinctoriale, et plus préférentiellement encore entre 5 et 30% en poids environ.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier entre 3 et 12 environ et de préférence entre 5 et 11 environ. Il peut être ajusté à la valeur désirée au moyen d'agents acidifiants ou alcalinisants ou de tampons habituellement utilisés en teinture des matières kératiniques.

Les agents acidifiants sont classiquement des acides minéraux ou organiques comme par exemple les acides chlorhydrique, orthophosphorique, sulfurique, les acides carboxyliques comme les acide acétique, tartrique, citrique, lactique et sulfonique.
Parmi les tampons, on peut citer par exemple, le phosphate diacide de potassium/hydroxyde de sodium.

Parmi les agents alcalinisants, on peut citer à titre d'exemple, l'ammoniaque, les carbonates alcalins, les alcanolamines telles que les mono- di- et tri-éthanolamines et leurs dérivés, les hydroxydes de sodium ou de potassium, et les composés de formule : 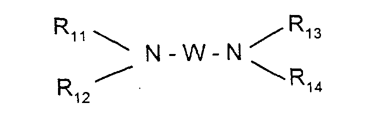
dans laquelle, W est un reste propylène éventuellement substitué par un groupement hydroxyle ou un radical alkyle en C₁-C₄ ; R₁₁ R₁₂, R₁₃ et R₁₄, simultanément ou indépendamment l'un de l'autre, représentent un atome d'hydrogène, un radical alkyle en C₁-C₆ ou hydroxyalkyle en C₁-C₆,

La composition appliquée sur les cheveux peut se présenter sous des formes diverses, telles que sous forme de liquide, de crème, de gel ou sous toute autre forme appropriée pour réaliser une teinture des matières kératiniques. En particulier, elle peut être conditionnée sous pression en flacon aérosol en présence d'un agent propulseur et former une mousse.

Un autre objet de la présente invention porte sur un procédé de teinture des fibres kératiniques, en particulier des fibres kératiniques humaines telles que les cheveux, par coloration directe, consistant à laisser agir une composition tinctoriale renfermant au moins une nitroaniline cationique monobenzénique de formule (I) sur les fibres kératiniques sèches ou humides. On peut utiliser la composition selon l'invention en tant que composition non rincée, c'est-à-dire qu'après application de la composition sur les fibres, on sèche sans rinçage intermédiaire.
Dans les autres modes d'application, on laisse agir la composition sur les fibres pendant un temps de pose variant entre 3 et 60 minutes environ, de préférence entre 5 et 45 minutes environ, on rince, éventuellement on lave, puis on rince à nouveau, et on sèche.

Des exemples concrets illustrant l'invention vont maintenant être donnés.

### EXEMPLES DE PREPARATION

### Exemple 1 : Préparation du composé de formule (I)₂ 3-[3-(4,5-dichloro-2-nitro-phénylamino)-propyl]-1 -méthyl-3H-imidazol-1-ium, méthylsulfate.

### ➢ 1^{ère} étape:

### synthèse du (4,5-dichloro-2-nitro-phényl)-(3-imidazol-1-yl-propyl)-amine

Sous agitation on a chauffé pendant 3 heures au reflux un mélange de 90,6g (0,4 mole) de 1,2,4-trichloro-5-nitro-benzène (RN 89-69-0), de 50,1 g (0,45 mole) de 3-imidazol-1-yl-propylamine (RN 5036-48-6) et de 62,7 ml (0,45 mole) de triéthylamine dans 100 ml de 1,2-diméthoxyéthane.
On a versé dans 1,51 d'eau glacée, essoré le précipité cristallisé, réempaté dans l'eau puis dans l'alcool isopropylique et séché sous vide à 40°C sur anhydride phosphorique.
On a obienu 54,5g de cristaux jaune orangé qui, après purification par recristallisation de l'alcool éthylique au reflux, ont fondu à 131°C (Kofler) et dont l'analyse élémentaire calculée pour C₁₃H₁₅N₄O₃Cl était :

| **%** | **C** | **H** | **N** | **O** | **Cl** |
|---|---|---|---|---|---|
| Calculé : | 45,73 | 3,84 | 17,78 | 0,15 | 22,50 |
| Trouvé : | 45,61 | 3,88 | 17,69 | 10,26 | 22,43 |

### ➢ 2^{éme} étape:

### quaternisation

On a réalisé la suspension de 4,7g (0,015 mole) de (4,5-dichloro-2-nitro-phényl)-(3-imidazol-1-yl-propyl)-amine obtenue ci-dessus à l'étape précédente et de 1,6 ml (0,017mole) de diméthylsulfate dans 75ml d'acétate d'éthyle et on a laissé 2 heures à température ambiante, sous agitation.
Le précipité cristallisé a été essoré, lavé plusieurs fois dans l'acétate d'éthyle, réempâté dans le minimum d'éthanol absolu et séché à 50°C sous vide.
On a obtenu 4,5g de cristaux jaunes qui ont fondu à 100°C (Kofler) et dont l'analyse élémentaire calculée pour C₁₄H₁₈N₄O₆SCl₂ était :

| **%** | **C** | **H** | **N** | **O** | **S** | **Cl** |
|---|---|---|---|---|---|---|
| Calculé : | 38,11 | 4,11 | 12,70 | 21,75 | 7,27 | 16,07 |
| Trouvé: | 38,14 | 4,16 | 12, 89 | 21,89 | 7,17 | 15,80 |

### Exemple 2 : Préparation du composé de formule (I)₁ 3-[3-(4-chloro-5-méthoxy-2-nitro-phénylamino)-propyl]-1-méthyl-3H-imidazol-1-ium, méthylsulfate.

### ➢ 1^{ère} étape:

### synthèse de la (4-chloro-5-méthoxy-2-nitro-phényl)-(3-imidazol-1-yl-propyl)-amine

Sous agitation, on a chauffé une heure au reflux une solution de 12,6g (0,04 mole) de (4,5-dichloro-2-nitro-phényl)-(3-imidazol-1-yl-propyl)-amine obtenue à la première étape de l'exemple 1 et de 0,08 mole de méthylate de sodium dans 100 ml de méthanol.
On a versé dans 500g d'eau glacée, essoré le précipité cristallisé, réempâté dans l'eau et séché sous vide sur anhydride phosphorique.
On a obtienu 9,5g de cristaux jaunes qui, après purification par recristallisation de l'éthanol au reflux, ont fondu à 178°C (Kofler) et dont l'analyse élémentaire calculée pour C₁₃H₁₅N₄O₃Cl était :

| **%** | **C** | **H** | **N** | **O** | **Cl** |
|---|---|---|---|---|---|
| Calculé : | 50,25 | 4,87 | 18,03 | 15,45 | 11,41 |
| Trouvé : | 49,79 | 4,98 | 17,44 | 16,36 | 11,23 |

### ➢ 2^{éme} étape:

### quaternisation

On a utilisé le mode opératoire décrit pour l'exemple 1 (2^{éme} étape).
A partir de 4,7g (0,015 mole) de (4-chloro-5-méthoxy-2-nitro-phényl)-(3-imidazol-1-yl-propyl)-amine obtenue ci-dessus à l'étape précédente et de 1,6 ml (0,017mole) de diméthylsulfate dans 75ml d'acétate d'éthyle on a obtenu 5,7g de cristaux jaunes qui ont fondu à 104°C (Kofler) et dont l'analyse élémentaire calculée pour C₁₅H₂₁N₄O₇SCl était :

| **%** | **C** | **H** | **N** | **O** | **S** | **Cl** |
|---|---|---|---|---|---|---|
| Calculé : | 41,24 | 4,85 | 12,82 | 25,64 | 7,34 | 8,12 |
| Trouvé : | 41,16 | 4,84 | 12,86 | 25,44 | 7,18 | 7,94 |

### Exemple 3 : Préparation du composé de formule (I)₃ 3-[3-(4-chloro-5-méthylsulfanyl-2-nitro-phénylamino)-propyl]-1-méthyl-3H-imidazol-1-ium, méthylsulfate.

### ➢ 1^{ère} étape:

### synthèse de la (4-chloro-5-méthylsulfanyl-2-nitro-phényl)-(3-imidazol-1-yl-propyl)-amine

Sous agitation, on a chauffé une heure au reflux, une solution de 12,6g (0,04 mole) de (4,5-dichloro-2-nitro-phényl)-(3-imidazol-1-yl-propyl)-amine obtenue à la première étape de l'exemple 1 et de 4,0g de thiométhylate de sodium dans 70 ml de 1,2-diméthoxyéthane.
On a versé dans 500g d'eau glacée, essoré le précipité cristallisé, réempâté dans l'eau et séché sous vide sur anhydride phosphorique.
On a obtenu 9,5g de cristaux jaune orangé qui, après purification par recristallisation du 1,2-diméthoxyéthane au reflux, ont fondu à 152°C (Kofler) et dont l'analyse élémentaire calculée pour C₁₃H₁₅N₄O₂SCl était :

| **%** | **C** | **H** | **N** | **O** | **S** | **Cl** |
|---|---|---|---|---|---|---|
| Calculé : | 47,78 | 4,63 | 17,14 | 9,79 | 9,81 | 10,85 |
| Trouvé : | 47,42 | 4,59 | 17,11 | 9,95 | 9,81 | 10,96 |

### ➢ 2^{ème}étape:

### quaternisation

On a utilisé le mode opératoire décrit pour l'exemple 1 (2^{ème} étape).
A partir de 4,9g (0,015 mole) de (4-chloro-5-méthylsulfanyl-2-nitro-phényl)-(3-imidazol-1-yl-propyl)-amine obtenué ci-dessus à l'étape précédente et de 1,6 ml (0,017mole) de diméthylsulfate dans 75ml d'acétate d'éthyle on a obtenu 5,3g de cristaux jaunes qui ont fondu à 102°C (Kofler) et dont l'analyse élémentaire calculée pour C₁₂H₂₁N₄O₆SCI était :

| **%** | **C** | **H** | **N** | **O** | **S** | **Cl** |
|---|---|---|---|---|---|---|
| Calculé : | 39,78 | 4,67 | 12,37 | 21,19 | 14,16 | 7,83 |
| Trouvé : | 39,77 | 4,59 | 12,45 | 21,23 | 13,95 | 7,96 |

### Exemple 4 : Préparation du composé de formule (I)₅ 1,3-diméthyl-2-(3-méthylamino-4-nitro-phénylsulfanyl)-3H-imidazol-1-ium, méthylsulfate.

### ➢1^{ère} étape:

### synthèse de la méthyl-[5-(1-méthyl-1H-imidazol-2-ylsulfanyl)-2-nitro-phényl]-amine

On a chauffé 4 heures à 120°C, un mélange de 9,33g (0,05 mole) de (5-chloro-2-nitro-phényl)-méthyl-amine (RN 35966-84-8), de 8,0g (0,07 mole) de 1-méthyl-1H-imidazole-2-thiol (RN 60-56-0) et de 10,4g (0,075 mole) de carbonate de potassium dans 30 ml de N-méthylpyrrolidone.
On a versé dans 250g d'eau glacée, essoré le précipté cristallisé, réempâté dans l'eau et séché sous vide sur anhydride phosphorique.
On a obtenu 12g de cristaux jaune foncé qui, après purification par recristallisation de l'éthanol au reflux, ont fondu à 140°C (Kofler) et dont l'analyse élémentaire calculée pour C₁₁H₁₂N₄O₂S était:

| **%** | **C** | **H** | **N** | **O** | **S** |
|---|---|---|---|---|---|
| Calculé : | 49,99 | 4,58 | 21,20 | 12,11 | 12,13 |
| Trouvé : | 49,81 | 4,65 | 21,20 | 12,35 | 12,18 |

### ➢2^{ème} étape:

### quaternisation

On a utilisé le mode opératoire décrit pour l'exemple 1 (2^{ème} étape).
A partir de 5,3g (0,02 mole) de méthyl-[5-(1-méthyl-1H-imidazol-2-ylsulfanyl)-2-nitro-phényl]-amine obtenue ci-dessus à l'étape précédente et de 2,09 ml (0,022mole) de diméthylsulfate dans 100ml d'acétate d'éthyle on a obtenu 6,0g de cristaux jaune orangé qui ont fondu à 170°C (Kofler) et dont l'analyse élémentaire calculée pour C₁₃H₁₈N₄O₆S était :

| **%** | **C** | **H** | **N** | **O** | **S** |
|---|---|---|---|---|---|
| Calculé: | 39,99 | 4,65 | 14,35 | 24,59 | 16,42 |
| Trouvé : | 39,98 | 4,79 | 14,40 | 24,72 | 16,33 |

### Exemple 5 : Préparation du composé de formule (I)₆ 1-{2-[5-(2-hydroxy-éthylamino)-2-méthyl-4-nitro-phénoxy]-éthyl}-3-méthyl-3H-imidazol-1-ium, bromure.

### ➢1^{ère} étape:

### synthèse de l'ester 2-chloroéthylique de l'acide (5-hydroxy-4-méthyl-2-nitro-phényl)-carbamique

On a chauffé au bain marie bouillant et sous agitation la suspension de 19,7g (0,118 mole) de 5-amino-2-méthyl-4-nitro-phénol (RN 37066-92-5) et de 6,6g (0,066 mole) de carbonate de calcium dans 60ml de dioxanne; on a ajouté en 5 minutes 14,5ml (0,14 mole) de 2-chloroéthyl-chloroformate (RN 627-11-2) et on a poursuivi le chauffage 1h ½ au bain marie bouillant.
On a filtré sur 500g d'eau glacée, essoré le précipité cristallisé, réempâté dans l'eau et séché sous vide sur anhydride phosphorique.
On a obtenu 26,9g de cristaux crème qui, après purification par recristallisation de l'acétate d'éthyle au reflux, ont fondu à 186°C (Kofler) et dont l'analyse élémentaire calculée pour C₁₀H₁₁N₂O₅Cl était :

| **%** | **C** | **H** | **N** | **O** | **Cl** |
|---|---|---|---|---|---|
| Calculé : | 43,73 | 4,04 | 10,20 | 29,12 | 12,91 |
| Trouvé : | 43,77 | 4,04 | 10,17 | 28,92 | 12,87 |

### ➢2^{ème} étape:

### synthèse du 5-(2-hydroxy-éthylamino)-2-méthyl-4-nitro-phénol

On a chauffé au bain marie bouillant pendant ¾ heure un mélange de 12,5g (0,0455 mole) d'ester 2-chloro-éthylique d'acide (5-hydroxy-4-méthyl-2-nitrophényl)-carbamique obtenu ci dessus à l'étape précédente et de 36ml de lessive de soude 5N.
On a refroidi, ajouté 50g de glace, acidifié avec de l'acide chlorhydrique aqueux, essoré le précipité cristallisé, réempâté dans l'eau et séché à 55°C sous vide et sur anhydride phosphorique.
On a obtenu 9,5g de cristaux brun rouge qui, après purification par recristallisation de l'éthanol au reflux, ont fondu à 204°C (Kofler) et dont l'analyse élémentaire calculée pour C₉H₁₂N₂O₄ était :

| **%** | **C** | **H** | **N** | **O** |
|---|---|---|---|---|
| Calculé : | 50,94 | 5,70 | 13,20 | 30,16 |
| Trouvé: | 50,96 | 5,74 | 13,40 | 30,08 |

### ➢ 3^{ème} étape:

### synthèse du 2-[5-(2-bromo-éthoxy)-4-méthyl-2-nitro-phénylamino]-éthanol

On a chauffé au bain marie bouillant et sous agitation, la suspension de 18,4g (0,0867 mole) de 5-(2-hydroxy-éthylamino)-2-méthyl-4-nitro-phénol obtenu ci dessus à l'étape précédente et de 6,2g (0,104 mole) d'oxyde de calcium dans 80ml de diméthylformamide; on a ajouté 22,5ml (0,26 mole) de 1,2-dibromo-éthane (RN 106-93-4) et poursuivi le chauffage 4h au bain marie bouillant.
On a filtré sur 150g d'eau glacée, essoré le précipité cristallisé, réempâté dans l'eau et séché sous vide à 45°C sur anhydride phosphorique.
On a obtenu 19,2g de cristaux orangé qui, après purification par chromatographie moyenne pression sur colonne de gel de silice (gradient d'heptane et d'acétate d'éthyle), ont fondu à 124°C (Kofler).

### ➢ 4^{éme} étape:

### quaternisation

On a réalisé la suspension de 5,5g (0,0172 mole) de 2-[5-(2-bromo-éthoxy)-4-méthyl-2-nitro-phénylamino]-éthanol obtenu à l'étape précédente et de 3,4g (0,04mole) de 1-méthyl-1 H-imidazole (RN 616-47-7) dans 20ml de toluène.
On a chauffé sous agitation au reflux du toluène pendant 4 heures, essoré bouillant et réempâté deux fois dans l'acétate d'éthyle puis dans l'éthanol absolu.
Après séchage à 40°C sous vide on a obtenu des cristaux jaune orangé (6,2g) de bromure de 1-{2-[5-(2-hydroxy-éthy)amino)-2-méthyl-4-nitro-phénoxyl-éthyl}-3-méthyl-3H-imidazol-1-ium fondant à 156°C (Kofler) et dont l'analyse élémentaire calculée pour C₁₅H₂₁N₄O₄Br était :

| **%** | **C** | **H** | **N** | **O** | **Br** |
|---|---|---|---|---|---|
| Calculé : | 44,90 | 5,28 | 13,96 | 15,95 | 19,91 |
| Trouvé: | 44,53 | 5,39 | 13,73 | 16,56 | 19,66 |

### Exemple 6 : Préparation du composé de formule (I)₄ 3-[3-(4-méthoxy-2-nitro-phénylamino)-propyl]-1-méthyl-3H-imidazol-1-ium, méthylsulfate, hydrate.

### ➢ 1^{ère} étape:

### synthèse de la (3-imidazol-1-yl-propyl)-(4-méthoxy-2-nitro-phényl)-amine

Sous agitation, on a chauffé pendant 4 heures à 100-110°C un mélange de 18,7g (0,1 mole) de 1-chloro-4-méthoxy-2-nitro-benzène (RN 10298-80-3) et de 84 ml de 3-imidazol-1-yl-propylamine (RN 5036-48-6).
On a versé sur 400g d'eau glacée; l'huile qui a décanté a été extraite à l'acétate d'éthyle; on a séché sur sulfate de sodium, filtré et évaporé à sec sous pression réduite.
On a obtenu une huile orangée (22,7g) qui, après purification par chromatographie sur une colonne de gel de silice (gradient d'heptane et d'acétate d'éthyle), a cristallisé (fusion à 95°C - Kofler) et dont l'analyse élémentaire calculée pour C₁₃H₁₆N₄O₃ était :

| **%** | **C** | **H** | **N** | **O** |
|---|---|---|---|---|
| Calculé : | 56,51 | 5,84 | 20,28 | 17,37 |
| Trouvé : | 56,25 | 5,86 | 20,48 | 17,28 |

### ➢ 2^{ème} étape:

### quaternisation

On a réalisé la suspension de 5,5g (0,02 mole) de (3-imidazol-1-yl-propyl)-(4-méthoxy-2-nitro-phényl)-amine obtenue ci-dessus à l'étape précédente et de 2,09 ml (0,022mole) de diméthylsulfate dans 100ml d'acétate d'éthyle et on a laissé 3 heures à température ambiante, sous agitation.
L'huile qui a décanté a été lavée plusieurs fois dans l'acétate d'éthyle et séchée à 50°C sous vide.
On a obtenu 7,7g d'huile orangée dont l'analyse élémentaire calculée pour C₁₅H₂₂N₄O₇S + H₂O était :

| **%** | **C** | **H** | **N** | **O** | **S** |
|---|---|---|---|---|---|
| Calculé : | 42,85 | 5,75 | 13,33 | 30,44 | 7,63 |
| Trouvé : | 42,54 | 5,84 | 13,32 | 29,78 | 8,43 |

### Exemple 7 : Préparation du composé de formule (I)₇ 3-{3-[4-(2-hydroxy-éthoxy)-2-nitro-phénylamino]-propyl}-1-méthyl-3H-imidazol-1-ium, méthylsulfate.

### ➢ 1^{ère} étape:

### synthèse du 2-[4-(3-imidazol-1-yl-propylamino)-3-nitro-phénoxy]-éthanol

On a utilisé le mode opératoire décrit pour l'exemple 6 (1^{ère} étape).
A partir de 21,7g (0,1 mole) de 2-(4-chloro-3-nitro-phénoxy)-éthanol
(RN 59820-27-8) et de 84 ml de 3-imidazol-1-yl-propylamine (RN 5036-48-6), on a obtenu 29,0g de cristaux orangé qui, après purification par chromatographie moyenne pression sur colonne de gel de silice (gradient d'heptane et d'acétate d'éthyle), ont fondu à 60°C (Kofler).

### > 2^{éme} étape:

### quaternisation

On a utilisé le mode opératoire décrit pour l'exemple 6 (2^{éme} étape).
A partir de 4,5g (0,0147 mole) de 2-[4-(3-imidazol-1-yl-propylamino)-3-nitro-phénoxy]-éthanol obtenu ci-dessus à l'étape précédente et de 1,54 ml (0,016mole) de diméthylsulfate dans 75ml d'acétate d'éthyle, on a obtenu 3,3g de cristaux orangé fondant à 133°C (Kofler) et dont l'analyse élémentaire calculée pour C₁₆H₂₄N₄O₈S était :

| **%** | **C** | **H** | **N** | **O** | **S** |
|---|---|---|---|---|---|
| Calculé : | 44,44 | 5,59 | 12,96 | 29,60 | 7,41 |
| Trouvé: | 44,38 | 5,55 | 12,92 | 29,77 | 7,32 |

### Exemple 8 : Préparation du composé de formule (I)₈ 3-méthyl-1-[2-(4-nitro-phénylamino)-éthyl]-3H-imidazol-1-ium, bromure.

On a utilisé le mode opératoire décrit pour l'exemple 5 (4^{ème} étape).
A partir de 49,0g (0,2 mole) de (2-bromo-éthyl)-(4-nitro-phényl)-amine (RN 55851-35-9) et de 19,8g (0,24mole) de 1-méthyl-1H-imidazole (RN 616-47-7) dans 200ml de toluène, on a obtenu des cristaux jaune pâle (62,3g) de bromure de 3-méthyl-1-[2-(4-nitro-phénylamino)-éthyl]-3H-imidazol-1-ium fondant à 214°C (Kofler) et dont l'analyse élémentaire calculée pour C₁₂H₁₅N₄O₂Br était :

| **%** | **C** | **H** | **N** | **O** | **Br** |
|---|---|---|---|---|---|
| Calculé : | 44,05 | 4,62 | 17,12 | 9,78 | 24,42 |
| Trouvé : | 44,14 | 4,57 | 17,03 | 9,78 | 24,37 |

### Exemple 9 : Préparation du composé de formule (I)₉ 1-méthyl-3-[3-(4-nitro-phénylamino)-propyl]-3H-imidazol-1-ium, méthylsulfate.

### ➢ 1^{ère} étape:

### synthèse de la (3-imidazol-1-yl-propyl)-(4-nitro-phényl)-amine

On a utilisé le mode opératoire décrit pour l'exemple 1 (1^{ère} étape).
A partir de 28,2g (0,2 mole) de 1-fluoro-4-nitro-benzène (RN 350-46-9) et de 31,3g (0,25 mole) de 3-imidazol-1-yl-propylamine (RN 5036-48-6), on a obtenu en ½ heure des cristaux jaune (36,3g) qui, après purification par recristallisation de l'éthanol au reflux, ont fondu à 124°C et dont l'analyse élémentaire calculée pour C₁₂H₁₄N₄O₂ était :

| **%** | **C** | **H** | **N** | **O** |
|---|---|---|---|---|
| Calculé : | 58,53 | 5,73 | 22,75 | 12,99 |
| Trouvé: | 58,17 | 5,75 | 22,67 | 13,45 |

### ➢ 2^{éme} étape:

### quaternisation

On a utilisé le mode opératoire décrit pour l'exemple 1 (2^{éme} étape).
A partir de 30,4g (0,123 mole) de (3-imidazol-1-yl-propyl)-(4-nitro-phényl)-amine obtenue ci-dessus à l'étape précédente et de 12,9 ml (0,135mole) de diméthylsulfate dans 600ml d'acétate d'éthyle, on a obtenu 37,6g de cristaux jaune qui ont fondu à 74°C (Kofler) et dont l'analyse élémentaire calculée pour C₁₄H₂₀N₄O₆S était :

| **%** | **C** | **H** | **N** | **O** | **S** |
|---|---|---|---|---|---|
| Calculé : | 45,15 | 5,41 | 15,04 | 25,78 | 8,61 |
| Trouvé: | 44,85 | 5,50 | 14,91 | 25,97 | 8,49 |

### Exemple 10 : Préparation du composé de formule (I)₁₀ chlorhydrate de 1-[3-(2-amino-5-nitro-phénoxy)-propyl]-3-méthyl-3H-imidazol-1-ium, chlorure.

### ➢1^{ère} étape:

### synthèse du N-[2-(3-chloro-propoxy)-4-nitro-phényl]-acetamide

Sous agitation, on a chauffé à 50°C un mélange de 98,1 g (0,5 mole) de N-(2-hydroxy-4-nitro-phényl)-acetamide (RN 121-88-0) et de 69,2g (0,5 mole) de carbonate de potassium dans 500 ml de diméthylformamide; puis on a ajouté 113,0g (1 mole) de 1,3-dichloro-propane (RN 142-28-9) et continué de chauffer à 50°C pendant une heure.
On a versé dans 4 litres d'eau glacée, essoré le précipité cristallisé, réempâté dans l'eau puis dans l'alcool isopropylique et séché sous vide à 40°C sur anhydride phosphorique.
On a obtenu 113,5g de cristaux beige qui, après purification par recristallisation de l'acétate d'isopropyle au reflux, ont fondu à 121°C .
L'analyse élémentaire calculée pour C₁₁H₁₃N₂O₄Cl était :

| **%** | **C** | **H** | **N** | **O** | **Cl** |
|---|---|---|---|---|---|
| Calculé : | 48,95 | 4,81 | 10,27 | 23,47 | 13,00 |
| Trouvé : | 48,33 | 4,84 | 10,14 | 22,67 | 12,87 |

### ➢ 2^{éme} étape:

### synthèse du 1-[3-(2-acétylamino-5-nitro-phénoxy)-propyl]-3-méthyl-3H-imidazol-1-ium, chlorure

On a utilisé le mode opératoire décrit pour l'exemple 5 (4^{ème} étape).
A partir de 27,2g (0,1 mole) de N-[2-(3-chloro-propoxy)-4-nitro-phényl]-acétamide obtenu ci-dessus à l'étape précédente et de 9,9g (0,12mole) de 1-méthyl-1H-imidazole (RN 616-47-7) dans 120ml de toluène, on a obtenu des cristaux jaune pâle (21,5g) de chlorure de 1-[3-(2-acétylamino-5-nitro-phénoxy)-propyl]-3-méthyl-3H-imidazol-1-ium fondant à 227°C (Kofler) et dont l'analyse élémentaire calculée pour C₁₅H₁₉N₄O₄Cl était :

| **%** | **C** | **H** | **N** | **O** | **Cl** |
|---|---|---|---|---|---|
| Calculé : | 50,78 | 5,40 | 15,79 | 18,04 | 9,99 |
| Trouvé: | 50,69 | 5,36 | 15,74 | 18,23 | 9,79 |

### ➢ 3^{éme} étape:

### désacétylation

On a chauffé une heure au reflux de l'alcool éthylique, 14,0g (0,039 mole) de chlorure de 1-[3-(2-acétylamino-5-nitro-phénoxy)-propyl]-3-méthyl-3H-imidazol-1-ium obtenu ci-dessus à l'étape précédente dans 50 ml d'éthanol absolu chlorhydrique (5N).
On a refroidi dans un bain de glace, essoré le précipité cristallisé, réempâté dans l'éthanol absolu et séché à 50°C sous vide et sur potasse.
On a obtenu des cristaux jaune pâle (10,0g) de chlorhydrate de 1-[3-(2-amino-5-nitro-phénoxy)-propyl]-3-méthyl-3H-imidazol-1-ium, chlorure fondant à 206°C (Kofler) et dont l'analyse élémentaire calculée pour C₁₃H₁₈N₄O₃Cl₂ était :

| **%** | **C** | **H** | **N** | **O** | **Cl** |
|---|---|---|---|---|---|
| Calculé : | 44,71 | 5,20 | 16,04 | 13,74 | 20,30 |
| Trouvé: | 44,43 | 5,20 | 15,78 | 13,52 | 19,90 |

### Exemple 11 : Préparation du composé de formule (I)₁₁ chlorure de 3-[3-(4,5-dichloro-2-nitro-phénylamino)-propyl]-1-(3-triméthylsilanyl-propyl)-3H-imidazol-1-ium, hydrate.

### > 1^{ème} étape:

### quaternisation

Sous agitation, on a chauffé 23 heures au reflux une solution de 15,76g (0,05 mole) de (4,5-dichloro-2-nitro-phényl)-(3-imidazol-1-yl-propyl)-amine obtenue à la première étape de l'exemple 1 et de 9,0g (0,06 mole) de 3-chloropropyltriméthylsilane (RN 2344-83-4) dans 50 ml de 2-méthyl 1-propanol. L'huile orangée obtenue après évaporation à sec du milieu réactionnel a été séchée à 45°C sous vide et sur anhydride phosphorique, puis elle a été reprise dans 50ml d'acétate d'éthyle.
Le composé attendu a cristallisé; après essorage, lavage à l'acétate d'éthyle et séchage, on a obtenu 12,2g de cristaux jaune-orangé fondant à 122-124°C (Kofler) et dont l'analyse élémentaire calculée pour C₁₈H₂₇N₄O₂Cl₃Si,H₂O était :

| **%** | **C** | **H** | **N** | **Cl** |
|---|---|---|---|---|
| Calculé : | 44,68 | 6,04 | 11,58 | 21,98 |
| Trouvé: | 44,98 | 5,95 | 11,67 | 21,93 |

### EXEMPLES DE COMPOSITIONS TINCTORIALES

### EXEMPLE 1:

On a préparé la composition de teinture suivante : *(toutes teneurs exprimées en grammes - M.A. désigne Matière Active)*

| | |
|---|---|
| Colorant de formule (I)_{II} | 0,484 |
| Hydroxyéthylcellulose vendue sous la dénomination NATROSOL 250 MR® par la société Aqualon | 0,72 |
| Alcool benzylique | 4 |
| Polyéthylèneglycol à 6 oxyde d'éthylène | 6 |
| Alkyl(C8-C10) polyglucoside en solution aqueuse à 60% M.A. vendu sous la dénomination ORAMIX CG 110® par la société Seppic | 4,5 M.A |
| Tampon phosphate pH 7 q.s.p | 100 |

On a appliqué la composition ci-dessus sur des mèches de cheveux gris naturels
ou permanentés à 90% de blancs et on a laissé poser pendant 20 minutes. Après rinçage à l'eau courante et séchage, les cheveux ont été teints dans une nuance jaune intense.

### EXEMPLES 2 à 7 :

On a préparé les six compositions de teinture suivantes :
*(toutes teneurs exprimées en grammes)*

| **EXEMPLE** | **2** | **3** | **4** | **5** | **6** | **7** |
|---|---|---|---|---|---|---|
| Composé de formule (I)₁ | 0,437 | | | | | |
| Composé de formule (I)₂ | | 0,441 | | | | |
| Composé de formule (I)₆ | | | 0,401 | | | |
| Composé de formule (I)₃ | | | | 0,453 | | |
| Composé de formule (I)₇ | | | | | 0,432 | |
| Composé de formule (I)₄ | | | | | | 0,402 |
| Monoéthyléther d'éthylène-glycol | 10 | 10 | 10 | 10 | 10 | 10 |
| Mélange alcool cétylstéarylique / lauryl sulfate de sodium commercialisé sous la dénomination SINNOWAX SX® par la société HENKEL | 2 | 2 | 2 | 2 | 2 | 2 |
| Alcool gras linéaire (C₁₃-C₁₅) oxyéthyléné (3OE) vendu sous la dénomination SYNPERONIC A3® par la société I.C.I | 3 | 3 | 3 | 3 | 3 | 3 |
| Alcool gras linéaire (C₁₃-C₁₅) oxyéthyléné (7OE) vendu sous la dénomination SYNPERONIC A7® par la société I.C.I | 2 | 2 | 2 | 2 | 2 | 2 |
| Bromure de triméthylcétylammonium | 1,5 | 1,5 | 1,5 | 1,5 | 1,5 | 1,5 |
| Monoéthanolamine qs pH | 8 | 8 | 8 | 8 | 8 | 8 |
| Eau déminéralisée qsp | 100 | 100 | 100 | 100 | 100 | 100 |

On a appliqué chacune des compositions ci-dessus sur des mèches de cheveux gris naturels à 90% de blancs et on a laissé poser pendant 20 minutes. Après rinçage à l'eau courante et séchage, les cheveux ont été teints dans une nuance qui est exprimée dans le tableau ci-dessous.

| | |
|---|---|
| Composition de l'exemple 2 | jaune |
| Composition de l'exemple 3 | jaune |
| Composition de l'exemple 4 | jaune |
| Composition de l'exemple 5 | jaune |
| Composition de l'exemple 6 | doré cuivré |
| Composition de l'exemple 7 | doré cuivré |

## Revendications

1. Utilisation à titre de colorants directs dans des, ou pour la fabrication de, compositions tinctoriales pour matières kératiniques en particulier pour fibres kératiniques humaines et notamment les cheveux, de nitroanilines cationiques monobenzéniques de formule (I) suivante et leurs sels d'addition avec un acide : 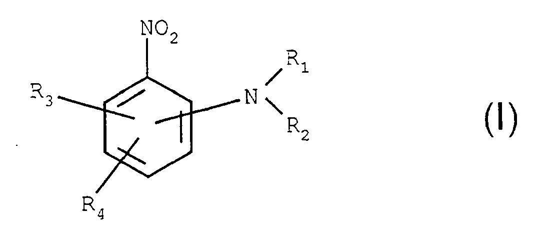
formule dans laquelle,
• **R**_{**1**}**, R**_{**2**}**,** qui peuvent être identiques ou différents, représentent un atome d'hydrogène ; un groupement Z défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁₋C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁₋C₆)carboxyalkyle en C₁-C₆ un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁₋C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁₋C₆)carbonyle, carbamyle, N-alkyl(C₁-C₆)carbamyle ou N,N-dialkyl(C₁₋C₆)carbamyle, atkyl(C₁-C₆)sulfonyle, formyle, trifluoroalkyl(C₁-C₆)carbonyle, alkyl(C₁-C₆)carboxy, thiocarbamyle, ou par un groupement Z défini ci-après ; ou pouvant former ensemble, avec l'atome d'azote auquel ils sont rattachés, un cycle à 5 ou 6 chaînons, carboné, ou contenant un ou plusieurs hétéroatomes ;
• **R**_{**3**}**, R**_{**4**}**,** qui peuvent être identiques ou différents, représentent un atome d'hydrogène ; un atome d'halogène ; un groupement Z défini ci-après ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁₋C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁₋C₆)carbonylalkyle(C₁-C₆) ; un radical N-alkyl(C₁-C₆)aminoalkyl(C₁₋C₆)carbonylalkyle(C₁-C₆) ; un radical N,N-dialkyl(C₁-C₆)aminoalkyl(C₁₋C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁₋C₆)aminosulfonylalkyle(C₁-C₆) ; un radical N,N-dialkyl(C₁₋C₆)aminosulfonylalkyle(C₁-C₆) ; un radical carbamyle ; un radical N-alkyl(C₁₋C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆); un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un radical aminoalkyle(C₁-C₆) dont l'alkyle est substitué ou non substitué par un ou plusieurs radicaux hydroxy et dont l'amine est non substituée ou substituée par un ou deux radicaux, identiques ou différents, choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁₋C₆)carbamyle ou N,N-dialkyl(C₁-C₆)carbamyle, alkyl(C₁-C₆)sulfonyle, formyle, trifluorcalkyl(C₁-C₆)carbonyle, alkyl(C₁-C₆)carboxy, thiocarbamyle, ou par un groupement Z défini ci-après ; un groupement OR₅ ou -SR₅ défini ci-après ; ou pouvant former ensemble, avec l'atome d'azote auquel ils sont rattachés, un cycle à 5 ou 6 chaînons, carboné, ou contenant un ou plusieurs hétéroatomes ;
• **R**_{**5**} désigne un atome d'hydrogène ; un radical alkyle en C₁-C₆; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ un radical alkyl(C₁₋C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylaikyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux, identiques ou différents, choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁₋C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou par un groupement Z défini ci-après ; ou pouvant former ensemble, avec l'atome d'azote auquel ils sont rattachés, un cycle à 5 ou 6 chaînons, carboné, ou contenant un ou plusieurs hétéroatomes ;
• **Z** est choisi parmi les groupements cationiques insaturés de formules (II) et (III) suivantes, et les groupements cationiques saturés de formule (IV) suivante : 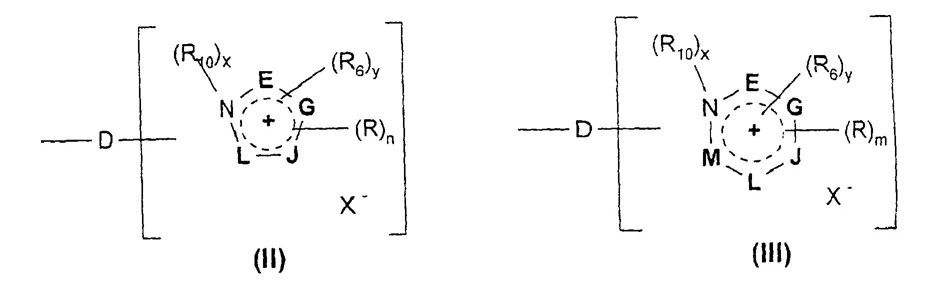 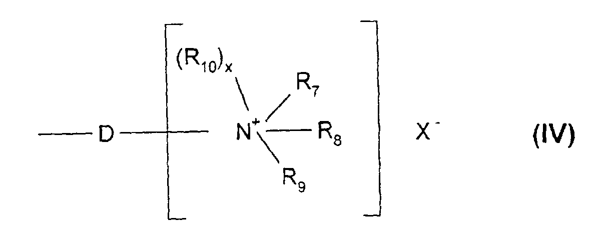
dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁₋C₆)thio, un radical amino, un radical amino protégé par un radical alkyl(C₁₋C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₆ représente un radical alkyle en C₁-C₆, un radical monohydroxyalkyle en C₁-C₆, un radical polyhydroxyalkyle en C₂-C₆, un radical cyanoalkyle en C₁₋C₆, un radical trialkyl(C₁-C₆)silanealkyle en C₁-C₆, un radical alcoxy(C₁₋C₆)alkyle en C₁-C₆, un radical carbamylalkyle C₁-C₆, un radical alkyl(C₁₋C₆)carboxyalkyle en C₁-C₆, un radical benzyle, un second groupement Z identique ou différent du premier groupement Z ;
• R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₇, R₈ et R₉ peuvent également former ensemble, avec l'atome d'azote auquel ils sont rattachés, un cycle saturé à 5 ou 6 chaînons, carboné, ou pouvant contenir un ou plusieurs hétéroatomes, tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁₋C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₇, R₈ et R₉ peut également représenter un second groupement Z, identique ou différent du premier groupement Z ;
• R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle
ou alkyl(C₁-C₆)sulfonyle ; un radical carboxyalkyle en C₁-C₆; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁₋C₆)sulfonylalkyle en C₁-C₆ ; un radical alkyl(C₁-C₆)cétoalkyle en C₁-C₆ ; un radical N-alkyl(C₁-C₆)carbamylaikyle en C₁-C₆ ; un radical N-alkyl(C₁₋C₆)sulfonamidoalkyle en C₁-C₆ ;
• x et y sont des nombres entiers égaux à 0 ou 1 ; avec les conditions suivantes :
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₆ est porté par l'atome d'azote du cycle insaturé ; ou bien
2) lorsqu'au moins un des sommets E, G, J et L représente un atome d'azote sur lequel le radical R₆ est fixé ;
- dans les groupements cationiques insaturés de formule (III) :
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₆ est porté par l'atome d'azote du cycle insaturé ;
- dans les groupements cationiques de formule (IV) :
- lorsque x = 0, alors le bras de liaison D est rattaché à l'atome d'azote portant les radicaux R₇ à R₉,
- lorsque x = 1, alors deux des radicaux R₇ à R₉ forment conjointement avec l'atome d'azote auquel ils sont rattachés un cycle saturé à 5 ou 6 chaînons tel que défini précédemment; et le bras de liaison D est porté par un atome de carbone dudit cycle saturé ;
• **X**⁻ représente un anion monovalent ou divalent,
**étant entendu :**
- que le nombre de groupements cationiques insaturés Z de formule (II), dans lesquels au moins un des sommets E, G, J et L représente un atome d'azote, est au moins égal à 1, et
- que, lorsque un et un seul des radicaux R₁ ou R₂ ou R₅ désigne un groupement Z dans lequel le bras de liaison D représente une chaîne alkyle comportant une fonction cétone, alors ladite fonction cétone n'est pas directement rattachée à l'atome d'azote du groupement NR₁R₂ ou à l'atome d'oxygène du groupement OR₅.

2. Utilisation selon la revendication 1, **caractérisée par le fait que** les cycles des groupements insaturés Z de formule (II) sont choisis parmi les cycles pyrrolique, imidazolique, pyrazolique, oxazolique, thiazolique et triazolique.

3. Utilisation selon la revendication 1, **caractérisée par le fait que** les cycles des groupements insaturés Z de formule (III) sont choisis parmi les cycles pyridinique, pyrimidinique, pyrazinique, oxazinique et triazinique.

4. Utilisation selon l'une quelconque des revendications précédentes, **caractérisée par le fait que** dans la formule (IV) deux des radicaux R₈, R₉ et R₁₀ forment un cycle pyrrolidinique, un cycle pipéridinique, un cycle pipérazinique ou un cycle morpholinique, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁₋C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁₋C₆)sulfonyle.

5. Utilisation selon l'une quelconque des revendications précédentes, **caractérisées par le fait que** X est choisi parmi un atome d'halogène, un hydroxyde, un hydrogènesulfate, ou un alkyl(C₁-C₆)sulfate.

6. Utilisation selon l'une quelconque des revendications précédentes, **caractérisées par le fait que** les nitroanilines cationiques monobenzéniques sont choisies parmi les composés suivants :
- le méthylsulfate du 3-[3-(4,5-dichloro-2-nitro-phénylamino)-propyl]-1-méthyl-3H-imidazol-1-ium,
- le méthylsulfate du 3-[3-(4-chloro-5-méthoxy-2-nitro-phénylamino)-propyl]-1-méthyl-3H-imidazol-1-ium,
- le méthylsulfate du 3-[3-(4-chloro-5-méthylsulfanyl-2-nitro-phénylamino)-propyl]-1-méthyl-3H-imidazol-1-ium,
- le méthylsulfate du 1,3-diméthyl-2-(3-méthylamino-4-nitro-phénylsulfanyl)-3H-imidazol-1-ium,
- le bromure du 1-{2-[5-(2-hydroxy-éthylamino)-2-méthyl-4-nitro-phénoxy]-éthyl}-3-méthyl-3-H-imidazol-1-ium,
- le méthylsulfate, hydrate, du 3-[3-(4-méthoxy-2-nitro-phénylamino)-propyl]-1-méthyl-3H-imidazol-1-ium,
- le méthylsulfate du 3-{3-[4-(2-hydroxyéthoxy)-2-nitro-phénylamino]-propyl}-1-méthyl-3H-imidazol-1-ium,
- le bromure du 3-méthyl-1-[2-(4-nitro-phénylamino)-éthyl]-3-H- imidazol-1-ium,
- le méthylsulfate du 1-méthyl-3-[3-(4-nitro-phénylamino)-propyl]-3-H-imidazol-1-ium,
- le chlorhydrate, chlorure du 1-[3-(2-amino-5-nitro-phénoxy)-propyl]-3méthyl-3H-imidazol-1-ium,
- le chlorure, hydrate de 3-[3-(4,5-dichloro-2-nitro-phénylamino)-propyl]-1-(3-triméthylsilanyl-propyl]-3-H-imidazol-1-ium,
- le bromure de 1-[3-(2-amino-5-nitro-phénoxy)-propyl]-2-méthyl-2H-pyrazol-1-ium,
- le méthylsulfate de 1-méthyl-3-[3-(2-nitro-phénylamino)-propyl]-3-H-imidazol-1-ium,
- le chlorure de 1-(3-chloro-propyl)-3-[3-(4-nitro-phénylamino)-propyl]-3-H-imidazol-1-ium,
- le chlorure de 1-(2-hydroxy-éthyl)-3-[3-(4-nitro-phénylamino)- propyl]-3-H-imidazol-1-ium,
- le méthylsulfate de 3-[3-(4-benzyloxy-2-nitro-phénylamino)- propyl]-1-méthyl-3-H-imidazol-1-ium,
- le méthylsulfate de 3-[3-(2-cyano-4-nitro-phénylamino)- propyl]-1-méthyl-3-H-imidazol-1-ium,
- le méthylsulfate de 1-méthyl-3-[3-(2-méthyl-4-nitro-phénylamino)- propyl]-3-H-imidazol-1-ium,
- le méthylsulfate de 3-[3-(2-fluoro-4-nitro-phénylamino)- propyl]-1-méthyl-3-H-imidazol-1-ium,
- le bromure de 1-[2-(2-méthoxy-4-nitro-phénylamino)-éthyl]-3-méthyl-3-H-imidazol-1-ium,
- le méthylsulfate de 3-[3-(3-hydroxy-4-nitro-phénylamino)- propyl]-1-méthyl-3-H-imidazol-1-ium,
- le méthylsulfate de 3-[3-(2-chloro-4-nitro-phénylamino)- propyl]-1-méthyl-3-H-imidazol-1-ium,
- le bromure de 2-méthyl-1-[2-(4-nitro-phénylamino)-éthyl]-2-H-pyrazol-1-ium,
- le bromure de 1-(3-bromo-2-hydroxy-propyl)-3-[3-(4-nitro-phénylamino)-propyl]-3-H-imidazol-1-ium,
- le dichlorure de 1-(3-triméthylammonium-2-hydroxy-propyl)-3-[3-(4-nitro-phénylamino)-propyl]-3H-imidazol-1-ium,
- le chlorure de diéthyl-(2-hydroxy-éthyl)-[4-(4-nitro-phénylamino)-pentyl]-ammonium,
- le méthylsulfate de 3-[3-(4-chloro-2-nitro-phénylamino)-propyl]-1-méthyl-3H-imidazol-1-ium,
- le méthylsulfate de 3-[3-(2-chloro-6-nitro-phénylamino)-propyl]-1-méthyl-3H-imidazol-1-ium,
- le chlorure de 1-méthyl-3-[2-(4-nitro-phénylamino)-butyl]-3H-imidazol-1-ium,
- le chlorure de 3-méthyl-1-[2-méthyl-2-(4-nitro-phénylamino)-propyl]-3H-imidazol-1-ium,
- le chlorure de 1-{2-[(2-(3-méthyl-3H-imidazol-1-ium)-éthyl)-(4-nitro-phényl)-amino]-éthyl}-3-méthyl-3H-imidazol-1-ium.

7. Utilisation selon la revendication 6, **caractérisées par le fait que** les nitroanilines cationiques monobenzéniques sont de formules (I)₁ à (I)₁₁ suivantes :

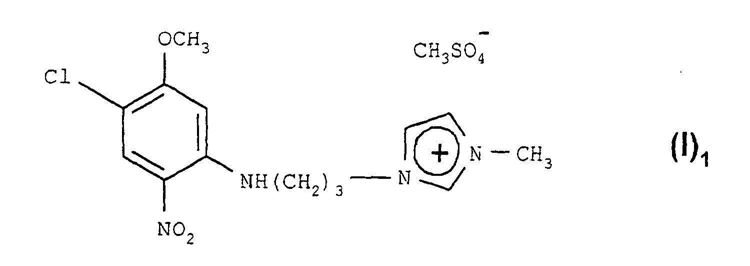

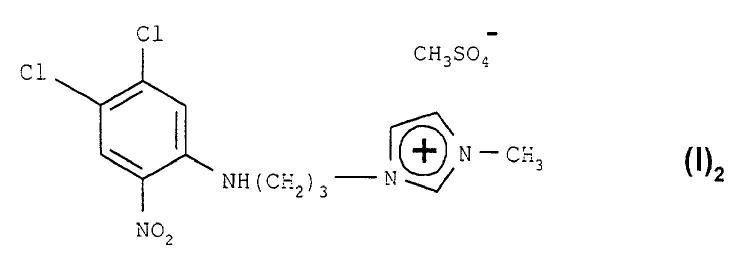

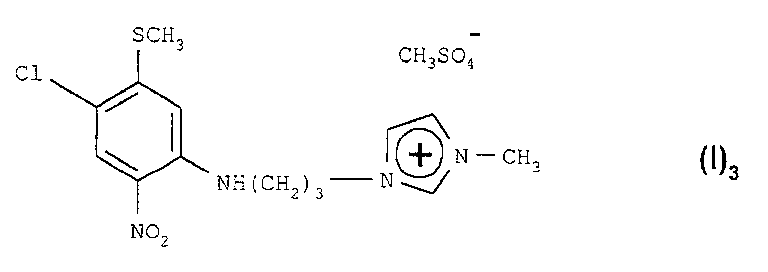

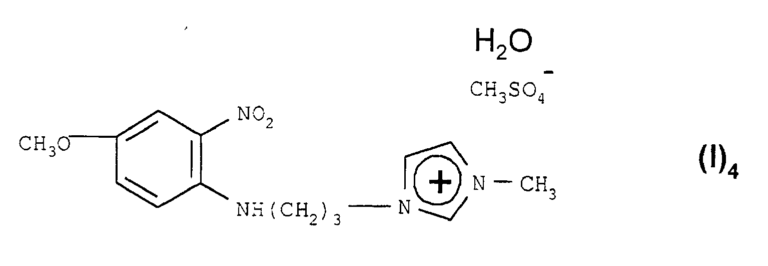

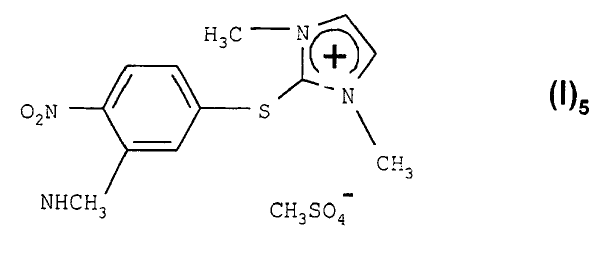

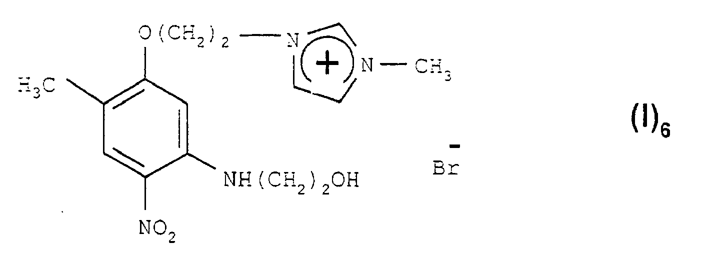

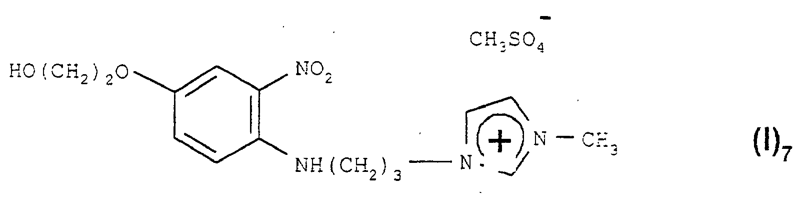

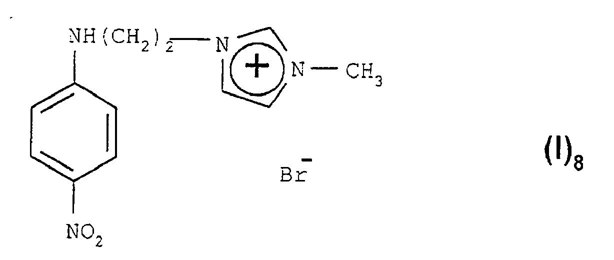

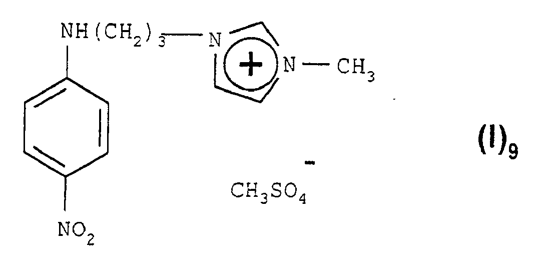

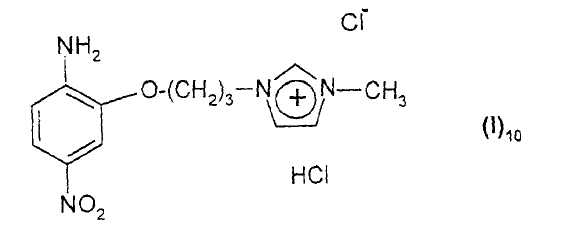

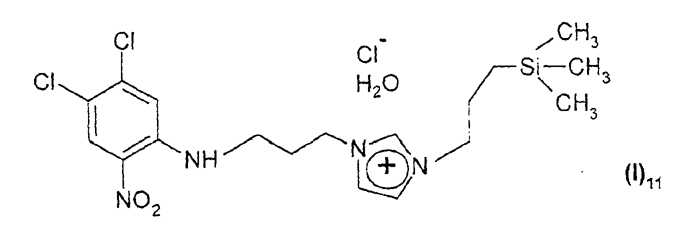

8. Utilisation selon l'une quelconque des revendications précédentes, **caractérisée par le fait que** les sels d'addition avec un acide des nitroanilines cationiques monobenzéniques de formule (I) sont choisis parmi les chlorhydrates, les bromhydrates, les sulfates, les citrates, les succinates, les tartrates, les lactates et les acétates.

9. Composition tinctoriale pour matières kératiniques, **caractérisée par le fait qu'**elle comprend, dans un milieu approprié pour la teinture, une quantité efficace d'au moins une nitroaniline cationique monobenzénique de formule (I) définie à l'une quelconque des revendications 1-7.

10. Composition de teinture directe pour fibres kératiniques humaines et notamment les cheveux, **caractérisée par le fait qu'**elle comprend, dans un milieu approprié pour la teinture, une quantité efficace d'au moins une nitroaniline cationique monobenzénique formule (I) définie à l'une quelconque des revendications 1-7.

11. Composition selon les revendications 9 ou 10, **caractérisée par le fait qu'**elle a un pH compris entre 3 et 12.

12. Composition selon l'une quelconque des revendications 9 à 11, **caractérisée par le fait que** les nitroanilines cationiques monobenzéniques de formule (1) sont présentes dans une concentration allant de 0,005 à 12 % en poids par rapport au poids total de la composition.

13. Composition selon la revendication 12, **caractérisée par le fait que** les nitroanilinés cationiques monobenzéniques de formule (1) sont présentes dans une concentration allant de 0,05 à 6 % en poids par rapport au poids total de la composition.

14. Composition selon l'une quelconque des revendications 9 à 13, **caractérisée par le fait que** le milieu approprié pour la teinture est un milieu aqueux constitué par de l'eau et/ou des solvants organiques, dans des proportions comprises entre 1 et 40% en poids par rapport au poids total de la composition.

15. Procédé de teinture des fibres kératiniques et en particulier des fibres kératiniques humaines et notamment les cheveux, par coloration directe, **caractérisé par le fait qu'**on applique une composition tinctoriale telle que définie à l'une quelconque des revendications 9 à 14, sur les fibres kératiniques sèches ou humides, et qu'on sèche ces fibres sans rinçage intermédiaire.

16. Procédé de teinture des fibres kératiniques et en particulier des fibres kératiniques humaines et notamment les cheveux, par coloration directe, **caractérisé par le fait qu'**on applique une composition tinctoriale telle que définie à l'une quelconque des revendications 9 à 14 sur les fibres kératiniques sèches ou humides, et qu'après avoir éventuellement laissé agir la composition sur les fibres pendant un temps de pose allant de 3 à 60 minutes, on rince les fibres, on les lave éventuellement, on les rince à nouveau puis on les sèche.

## Patentansprüche

1. Verwendung von kationischen Nitroanilinen auf Monobenzolbasis der folgenden Formel (I) und ihren Additionssalzen mit einer Säure als Direktfarbstoffe in Farbmittelzusammensetzungen zum Färben von Keratinfasern und insbesondere menschlichen Keratinfasern, besonders Haaren, oder für die Herstellung solcher Farbmittelzusammensetzungen: 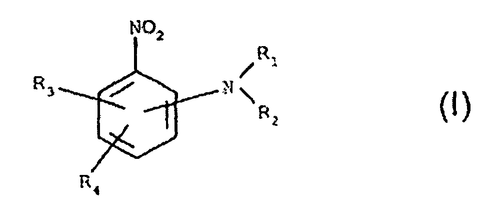
wobei in der Formel bedeuten:
- die Gruppen **R**_{**1**} und **R**_{**2**}**,** die gleich oder verschieden sein können, ein Wasserstoffatom; eine nachfolgend definierte Gruppe Z; Alkyl(C₁₋₆); Monohydroxyalkyl(C₁₋₆); Polyhydroxyalkyl(C₂₋₆); Alkoxy(C₁₋₆)alkyl(C₁₋₆); Aryl; Benzyl; Cyanoalkyl(C₁₋₆); Carbamoylalkyl(C₁₋₆); N-Alkyl(C₁₋₆)carbamoyl-alkyl(C₁₋₆); N,N-Dialkyl(C₁₋₆)carbamoylalkyl(C₁₋₆); Thiocarba-moylalkyl(C₁₋₆); Trifluoralkyl(C₁₋₆); Sulfoalkyl(C₁₋₆); Alkyl(C₁₋₆)carboxyalkyl(C₁₋₆); Alkyl(C₁₋₆)sulfinylalkyl(C₁₋₆); Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)carbonylalkyl(C₁₋₆); Aminoalkyl(C₁₋₆), dessen Alkylgruppe unsubstituiert vorliegt oder mit einer oder mehreren Hydroxygruppen substituiert ist; Aminoalkyl(C₁₋₆), dessen Alkylgruppe unsubstituiert vorliegt oder mit einer oder mehreren Hydroxygruppen substituiert ist und dessen Aminogruppe mit einer oder zwei Gruppen, die gleich oder verschieden sind und die unter den Gruppen Alkyl, Monohydroxyalkyl(C₁₋₆), Polyhydroxyalkyl(C₂₋₆), Alkyl(C₁₋₆)carbonyl, Carbamoyl, N-Alkyl(C₁₋₆)carbamoyl oder N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy, Thiocarbamoyl ausgewählt sind, oder mit einer nachstehend definierten Gruppe Z substituiert ist; oder sie können gemeinsam mit dem Stickstoffatom, an das sie gebunden sind, einen 5-oder 6-gliedrigen Ring auf Kohlenstoffbasis oder mit einem oder mehreren Heteroatomen bilden;
- die Gruppen **R**_{**3**} und **R**_{**4**}**,** die gleich oder verschieden sein können, ein Wasserstoffatom; ein Halogenatom; eine nachstehend definierte Gruppe Z; Alkyl(C₁₋₆)carbonyl; Aminoalkyl(C₁₋₆)carbonyl; N-Z-aminoalkyl(C₁₋₆)carbonyl; N-Alkyl(C₁₋₆)aminoalkyl(Ci-6)carbonyl; N,N-Dialkyl(C₁₋₆)aminoalkyl(C₁₋₆)carbonyl; Aminoalkyl(C₁₋₆)carbonylalkyl(Ci₁₋₆); N-Z-Aminoalkyl(C₁₋₆)carbonylalkyl(C₁₋₆); N-Alkyl(C₁₋₆)aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Z-Aminosulfonyl; N-Alkyl(C₁₋₆)aminosulfonyl; N,N-Dialkyl(C₁₋₆)aminosulfonyl; Aminosulfonylalkyl (C₁₋₆); N-Z-Aminosulfonylalkyl(C₁₋₆); N-Alkyl(C₁₋₆)aminosulfonylalkyl(C₁₋₆); N,N-Dialkyl(C₁₋₆)-aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Alkyl(C₁₋₆); Monohydroxy-alkyl(C₁₋₆); Polyhydroxyalkyl(C₂₋₆); Alkoxy((C₁₋₆)alkyl(C₁₋₆); Trifluoralkyl(C₁₋₆); Cyano, Aminoalkyl(C₁₋₆), dessen Alkylgruppe unsubstituiert vorliegt oder mit einer oder mehreren Hydroxygruppen substituiert ist und dessen Aminogruppe unsubstituiert vorliegt oder mit einer oder zwei Gruppen substituiert ist, die gleich oder verschieden sind und ausgewählt sind unter den Gruppen Alkyl(C₁₋₆), Mono-hydroxyalkyl(C₁₋₆), Polyhydroxyalkyl(C₂₋₆), Alkyl(C₁₋₆)-carbonyl, Carbamoyl, N-Alkyl(C₁₋₆)carbamoyl oder N,N-Dialkyl(C₁₋₆)carbamoyl, Alkyl(C₁₋₆)sulfonyl, Formyl, Trifluo-ralkyl(C₁₋₆)carbonyl, Alkyl(C₁₋₆)carboxy, Thiocarbamoyl oder mit einer nachfolgend definierten Gruppe Z; einer nachfolgend definierten Gruppe OR₅ oder -SR₅; oder sie können gemeinsam mit dem Stickstoffatom, an das sie gebunden sind, einen 5- oder 6-gliedrigen Ring auf Kohlenstoffbasis oder mit einem oder mehreren Heteroatomen bilden;
- **R**_{**5**} ein Wasserstoffatom; Alkyl(C₁₋₆); Monohydroxyalkyl(C₁₋₆); Polyhydroxyalkyl(C₂₋₆); eine Gruppe Z; Alkoxy(C₁₋₆)alkyl(C₁₋₆); Aryl; Benzyl; Carboxyalkyl(C₁₋₆); Alkyl(C₁₋₆)carboxyalkyl(C₁₋₆); Cyanoalkyl(C₁₋₆); Carbamoylalkyl(C₁₋₆); N-Alkyl(C₁₋₆)-carbamoylalkyl(C₁₋₆); N,N-Dialkyl(C₁₋₆)carbamoylalkyl(C₁₋₆); Trifluoralkyl(C₁₋₆); Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinyl-alkyl(C₁₋₆); Alkyl(C₁₋₆)sulfonylalkyl(C₁₋₆); Alkyl(C₁₋₆)carbonyl-alkyl(C₁₋₆); Aminoalkyl(C₁₋₆), dessen Alkylgruppe unsubstituiert vorliegt oder mit einer oder mehreren Hydroxygruppen substituiert ist; Aminoalkyl(C₁₋₆), dessen Alkylgruppe unsubstituiert vorliegt oder mit einer oder mehreren Hydroxygruppen substituiert ist und dessen Aminogruppe mit einer oder zwei Gruppen substituiert ist, die gleich oder verschieden sind und ausgewählt sind unter Alkyl(C₁₋₆), Mono-hydroxyalkyl(C₁₋₆), Polyhydroxyalkyl(C₂₋₆), Alkyl(C₁₋₆)-carbonyl, Formyl, Trifluoralkyl(C₁₋₆)carbonyl, Alkyl(C₁₋₆)-carboxy, Carbamoyl, N-Alkyl(C₁₋₆)carbamoyl, N,N-Dialkyl(C₁₋₆)carbamoyl, Thiocarbamoyl, Alkyl(C₁₋₆)sulfonyl oder mit einer nachfolgend definierten Gruppe Z; oder sie kann gemeinsam mit dem Stickstoffatom, an das sie gebunden ist, einen 5- oder 6-gliedrigen Ring auf Kohlenstoffbasis oder mit einem oder mehreren Heteroatomen bilden;
- die Gruppe Z ist unter den ungesättigten kationischen Gruppen der folgenden Formeln (II) und (III) und den gesättigten kationischen Gruppen der folgenden Formel (IV) ausgewählt: 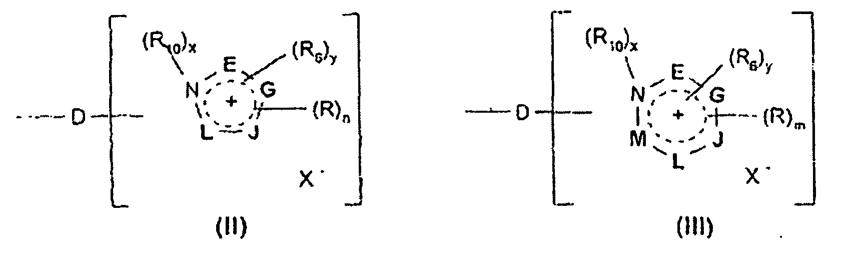 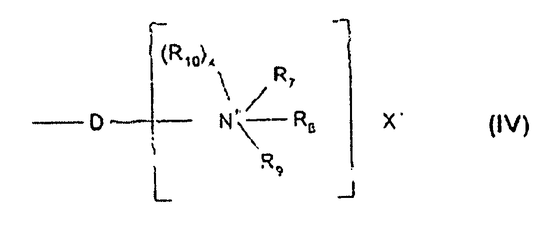
worin bedeuten:
- D eine Verbindungsgruppe, die eine geradkettige oder verzweigte Alkylgruppe mit vorzugsweise 1 bis 14 Kohlenstoffatomen bedeutet, die durch ein oder mehrere Heteroatome, wie Sauerstoff, Schwefel oder Stickstoff unterbrochen und mit einer oder mehreren Hydroxygruppen oder C₁₋₆-Alkoxygruppen substituiert sein kann und eine oder mehrere Ketofunktionen tragen kann;
- die Ringbestandteile E, G, J, L und M, die gleich oder verschieden sind, ein Kohlenstoffatom, ein Sauerstoffatom, ein Schwefelatom oder ein Stickstoffatom;
- n 0 oder eine ganze Zahl von 1 bis 4;
- m 0 oder eine ganze Zahl von 1 bis 5;
- die Gruppen R, die gleich oder verschieden sind, eine zweite Gruppe Z, die mit der ersten Gruppe Z identisch oder von ihr verschieden ist, ein Halogenatom, Hydroxy, Alkyl(C₁₋₆), Monohydroxyalkyl(C₁₋₆), Polyhydroxyalkyl(C₂₋₆), Nitro, Cyano, Cyanoalkyl(C₁₋₆), Alkoxy(C₁₋₆), Trialkyl(C₁₋₆)silanalkyl(C₁₋₆), Amido, Aldehyd, Carboxy, Alkylcarbonyl(C₁₋₆), Thio, Thioalkyl(C₁₋₆), Alkyl(C₁₋₆)thio, Amino, eine Aminogruppe, die mit Alkyl(C₁₋₆)carbonyl, Carbamoyl oder Alkyl(C₁₋₆)sulfonyl geschützt ist; NHR" oder NR"R"', worin die Gruppen R" und R"', die gleich oder verschieden sind, Alkyl(C₁₋₆), Monohydroxyal-kyl(C₁₋₆) oder Polyhydroxyalkyl(C₂₋₆) bedeuten;
- R₆ Alkyl(C₁₋₆), Monohydroxyalkyl(C₁₋₆), Polyhydroxyalkyl(C₂₋₆), Cyanoalkyl(C₁₋₆), Trialkyl(C₁₋₆)silanalkyl(C₁₋₆), Alkoxy(C₁₋₆)alkyl(C₁₋₆), Carbamoylalkyl(C₁-₆), Alkyl(C₁₋₆)carboxyalkyl(C₁₋₆), Benzyl, eine zweite Gruppe Z, die mit der ersten Gruppe Z identisch ist oder von ihr verschieden ist;
- R₇, R₈ und R₉, die gleich oder verschieden sind, Alkyl(C₁₋₆), Monohydroxyalkyl(C₁₋₆), Polyhydroxyalkyl(C₂₋₆), Alkoxy(C₁₋₆)alkyl(C₁₋₆), Cyanoalkyl(C₁₋₆), Aryl, Benzyl, Amidoalkyl(C₁₋₆), Trialkyl(C₁₋₆)silanalkyl(C₁₋₆) oder Aminoalkyl(C₁₋₆), dessen Aminogruppe mit Alkyl(C₁₋₆)carbonyl, Carbamoyl oder Alkyl(C₁₋₆)sulfonyl geschützt ist; wobei zwei der Gruppen R₇, R₈ und R₉ gemeinsam mit dem Stickstoffatom, an das sie gebunden sind, einen gesättigten, 5- oder 6-gliedrigen Ring auf Kohlenstoffbasis oder mit einem oder mehreren Heteroatomen bilden kann, wie beispielsweise Pyrrolidin, Piperidin, Piperazin oder Morpholin, wobei der Ring unsubstituiert vorliegen oder substituiert sein kann mit einem Halogenatom, Hydroxy, Alkyl(C₁₋₆), Mono-hydroxyalkyl(C₁₋₆), Polyhydroxyalkyl(C₂₋₆), Nitro, Cyano, Cyanoalkyl(C₁₋₆), Alkoxy(C₁₋₆), Trialkyl(C₁₋₆)silan-alkyl(C₁₋₆), Amido, Aldehydo, Carboxy, Ketoalkyl(C₁₋₆), Thio, Thioalkyl(C₁₋₆), Alkyl(C₁₋₆)thio, Amino, Amino, das mit Alkyl(C₁₋₆)carbonyl, Carbamoyl oder Alkyl(C₁₋₆)-sulfonyl geschützt ist;
wobei eine der Gruppen R₇, R₈ und R₉ auch eine zweite Gruppe Z bedeuten kann, die mit der ersten Gruppe Z identisch oder von ihr verschieden ist;
- R₁₀ Alkyl(C₁₋₆); Monohydroxyalkyl(C₁₋₆); Polyhydroxyalkyl(C₂₋₆); Aryl; Benzyl; Aminoalkyl(C₁₋₆), Aminoalkyl(C₁₋₆), dessen Aminogruppe mit Alkyl(C₁₋₆)carbonyl, Carbamoyl oder Alkyl(C₁₋₆)sulfonyl geschützt ist; Carboxyalkyl(C₁₋₆); Cyanoalkyl(C₁₋₆); Carbamoylalkyl(C₁₋₆); Trifluoralkyl(C₁₋₆); Trialkyl(C₁₋₆)silanalkyl(C₁₋₆); Sulfonamidoalkyl(C₁₋₆); Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
- x und y 0 oder 1; mit den folgenden Maßgaben:
- in den ungesättigten kationischen Gruppen der Formel (II):
- die Verbindungsgruppe D ist an das Stickstoffatom gebunden, wenn x = 0,
- die Verbindungsgruppe D ist an einen der Ringbestandteile E, G, J oder L gebunden, wenn x = 1,
- y kann nur den Wert 1 annehmen, wenn:
1) die Ringbestandteile E, G, J und L gleichzeitig ein Kohlenstoffatom bedeuten und die Gruppe R₆ von dem Stickstoffatom des ungesättigten Rings getragen wird; oder
2) mindestens einer der Ringbestandteile E, G, J und L ein Stickstoffatom bedeutet, an das die Gruppe R₆ gebunden ist;
- in den ungesättigten kationischen Gruppen der Formel (III):
- die Verbindungsgruppe D ist an das Stickstoffatom gebunden, wenn x = 0,
- die Verbindungsgruppe D ist an einen Ringbestandteil E, G, J, L oder M gebunden, wenn x = 1,
- y nur den Wert 1 annehmen kann, wenn mindestens einer der Ringbestandteile E, G, J, L und M ein zweiwertiges Atom bedeutet und die Gruppe R₆ von dem Stickstoffatom des ungesättigten Rings getragen wird;
- in den kationischen Gruppen der Formel (IV):
- die Verbindungsgruppe ist an das Stickstoffatom gebunden, das die Gruppen R₇ bis R₉ trägt, wenn x = 0,
- zwei der Gruppen R₇ bis R₉ bilden gemeinsam mit dem Stickstoffatom, an das sie gebunden sind, einen gesättigten 5- oder 6-gliedrigen Ring, wie er oben definiert wurde, und die Verbindungsgruppe D wird von einem Kohlenstoffatom des gesättigten Rings getragen, wenn x = 1;
- **X**⁻ ein einwertiges oder zweiwertiges Anion;
**mit der Maßgabe, dass:**
- die Anzahl der ungesättigten kationischen Gruppen Z der Formel (II), bei denen mindestens einer der Ringbestandteile E, G, J und L ein Stickstoffatom bedeutet, mindestens 1 ist, und
- wenn eine und nur eine der Gruppen R₁ oder R₂ oder R₅ eine Gruppe Z bedeutet, bei der die Verbindungsgruppe D eine Alkylgruppe ist, die eine Ketofunktion aufweist, die Ketonfunktion nicht direkt an das Stickstoffatom der Gruppe NR₁R₂ oder das Sauerstoffatom der Gruppe OR₅ gebunden ist.

2. Verwendung nach Anspruch 1, **dadurch gekennzeichnet, dass** die Ringe der ungesättigten Gruppen Z der Formel (II) unter Pyrrol, Imidazol, Pyrazol, Oxazol, Thiazol und Triazol ausgewählt sind.

3. Verwendung nach Anspruch 1, **dadurch gekennzeichnet, dass** die Ringe der ungesättigten Gruppen Z der Formel (III) unter Pyridin, Pyrimidin, Pyrazin, Oxazin und Triazin ausgewählt sind.

4. Verwendung nach einem der vorhergehenden Ansprüche, **dadurch gekennzeichnet, dass** in der Formel (IV) zwei der Gruppen R₈, R₉ und R₁₀ einen Pyrrolidinring, Piperidinring, Piperazinring oder Morpholinring bilden, wobei der Ring unsubstituiert vorliegen oder substituiert sein kann mit einem Halogenatom, Hydroxy, Alkyl(C₁₋₆), Monohydroxyalkyl(C₁₋₆), Polyhydroxyalkyl(C₂₋₆), Nitro, Cyano, Cyanoalkyl(C₁₋₆), Alkoxy(C₁₋₆), Trialkyl(C₁₋₆)silanalkyl(C₁₋₆), Amido, Aldehydo, Carboxy, Alkylcarbonyl(C₁₋₆), Thio, Thioalkyl(C₁₋₆), Alkyl(C₁₋₆)thio, Amino, einer Aminogruppe, die mit Alkyl(C₁₋₆)carbonyl, Carbamoyl oder Alkyl(C₁₋₆)sulfonyl geschützt ist

5. Verwendung nach einem der vorhergehenden Ansprüche, **dadurch gekennzeichnet, dass** X⁻ unter einem Halogenatom, Hydroxid, Hydrogensulfat oder einem Alkyl(C₁₋₆)sulfat ausgewählt ist.

6. Verwendung nach einem der vorhergehenden Ansprüche, **dadurch gekennzeichnet, dass** die kationischen Nitroaniline auf Monobenzolbasis unter den folgenden Verbindungen ausgewählt sind:
- 3-[3-(4,5-Dichlor-2-nitrophenylamino)-propyl]-1-methyl-3H-imidazol-1-ium-methylsulfat,
- 3-[3-(4-Chlor-5-methoxy-2-nitrophenylamino)-propyl]-1-methyl-3H-imidazol-1 -ium-methylsulfat,
- 3-[3-(4-Chlor-5-methylsulfanyl-2-nitrophenylamino)-propyl]-1-methyl-3H-imidazol-1-ium-methylsulfat,
- 1,3-Dimethyl-2-(3-methylamino-4-nitro-phenylsulfanyl)-3H-imidazol-1-ium-methylsulfat,
- 1-{2-[5-(2-Hydroxyethylamino)-2-methyl-4-nitro-phenoxy]-ethyl}-3-methyl-3-H-imidazol-1-ium-bromid,
- 3-[3-(4-Methoxy-2-nitrophenylamino)-propyl]-1-methyl-3H-imidazol-1-ium-methylsulfat, Hydrat,
- 3-{3-[4-(2-Hydroxyethoxy)-2-nitrophenylamino]-propyl}-1-methyl-3H-imidazol-1-ium-methylsulfat,
- 3-Methyl-1-[2-(4-nitrophenylamino)-ethyl]-3-H-imidazol-1-ium-bromid,
- 1-Methyl-3-[3-(4-nitrophenylamino)-propyl]-3-H-imidazol-1-ium-methylsulfat,
- 1-[3-(2-Amino-5-nitro-phenoxy)-propyl]-3-methyl-3H-imidazol-1-ium-chlorid-Hydrochlorid,
- 3-[3-(4,5-Dichlor-2-nitrophenylamino)-propyl]-1-(3-trimethylsilanyl-propyl]-3-H-imidazol-1-ium-chlorid, Hydrat,
- 1-[3-(2-Amino-5-nitro-phenoxy)-propyl]-2-methyl-2H-pyrazol-1-ium-bromid,
- 1-Methyl-3-[3-(2-nitrophenylamino)-propyl]-3-H-imidazol-1-ium-methylsulfat,
- 1-(3-Chlorpropyl)-3-[3-(4-nitrophenylamino)-propyl]-3-H-imidazol-1-ium-chlorid,
- 1-(2-Hydroxyethyl)-3-[3-(4-nitrophenylamino)-propyl]-3-H-imidazol-1-ium-chlorid,
- 3-[3-(4-Benzyloxy-2-nitrophenylamino)-propyl]-1-methyl-3-H-imidazol-1-ium-methylsulfat,
- 3-[3-(2-Cyano-4-nitrophenylamino)-propyl]-1-methyl-3-H-imidazol-1-ium-methylsulfat,
- 1-Methyl-3-[3-(2-methyl-4-nitrophenylamino)-propyl]-3-H-imidazol-1-ium-methylsulfat,
- 3-[3-(2-Fluor-4-nitrophenylamino)-propyl]-1-methyl-3-H-imidazol-1-ium-methylsulfat,
1- [2-(2-Methoxy-4-nitrophenylamino)-ethyl]-3-methyl-3-H-imidazol-1 -ium-bromid,
- 3-[3-(3-Hydroxy-4-nitrophenylamino)-propyl]-1-methyl-3-H-imidazol-1-ium-methylsulfat,
- 3-[3-(2-Chlor-4-nitrophenylamino)-propyl]-1-methyl-3-H-imidazol-1-ium-methylsulfat,
- 2-Methyl-1-[2-(4-nitrophenylamino)-ethyl]-2-H-pyrazol-1-ium-bromid,
- 1-(3-Brom-2-hydroxypropyl)-3-[3-(4-nitrophenylamino)-propyl]-3-H-imidazol-1-ium-bromid,
- 1-(3-Trimethylammonium-2-hydroxypropyl)-3-[3-(4-nitrophenylamino)-propyl]-3H-imidazol-1-ium-dichlorid,
- Diethyl-(2-hydroxyethyl)-[4-(4-nitrophenylamino)-pentyl]-ammonium-chlorid,
- 3-[3-(4-Chlor-2-nitro-phenylamino)-propyl]-1-methyl-3H-imidazol-1-ium-methylsulfat,
- 3-[3-(2-Chlor-6-nitro-phenylamino)-propyl]-1-methyl-3H-imidazol-1-ium-methylsulfat,
- 1-Methyl-3-[2-(4-nitrophenylamino)-propyl]-3H-imidazol-1-ium-chlorid,
- 3-Methyl-1-[2-methyl-2-(4-nitrophenylamino)-butyl]-3H-imidazol-1-ium-chlorid,
- 1-{2-[(2-(3-Methyl-3H-imidazol-1-ium)-ethyl)-(4-nitrophenyl)-amino]-ethyl}-3-methyl-3H-imidazol-1-ium-chlorid.

7. Verwendung nach Anspruch 6, **dadurch gekennzeichnet, dass** die kationischen Nitroaniline auf Monobenzolbasis den folgenden Formeln (I)₁ bis (I)₁₁ entsprechen:

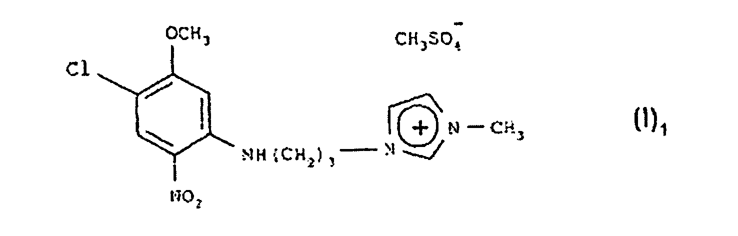

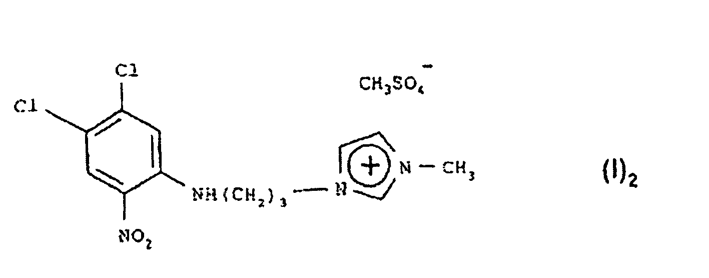

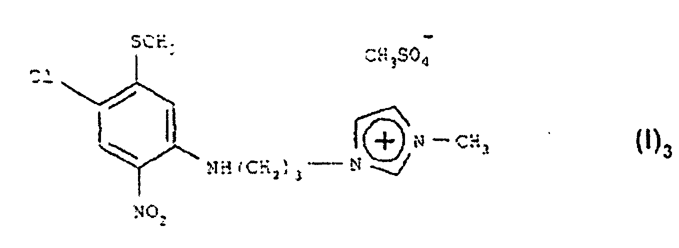

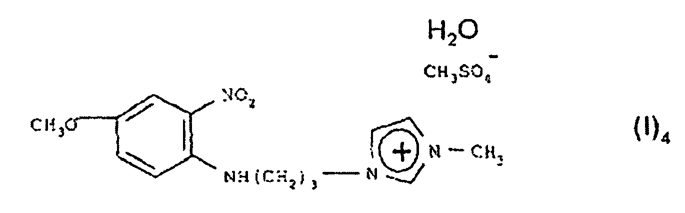

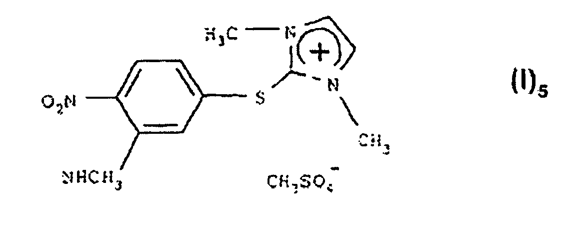

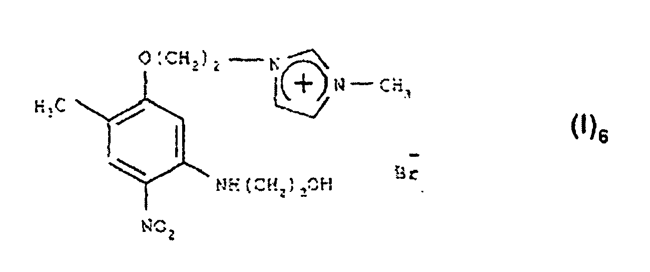

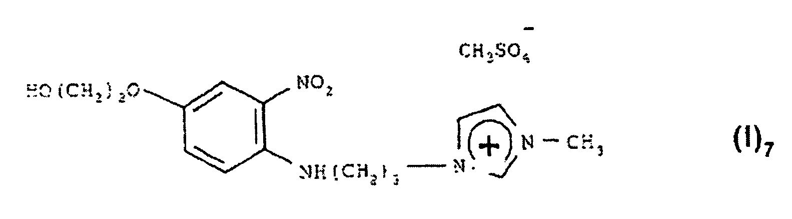

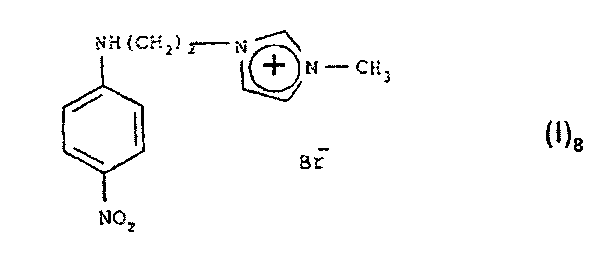

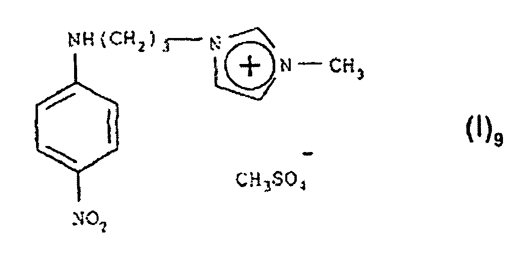

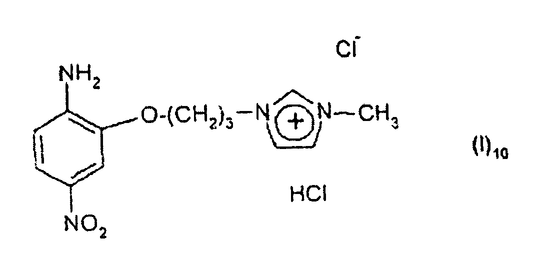

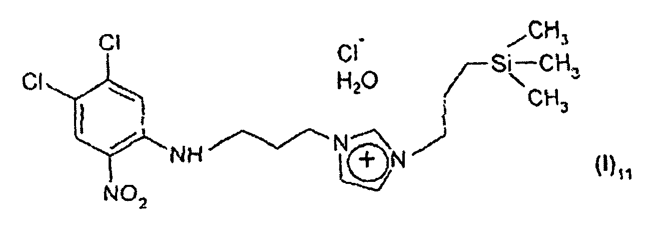

8. Verwendung nach einem der vorhergehenden Ansprüche, **dadurch gekennzeichnet, dass** die Additionssalze der kationischen Nitroaniline auf Monobenzolbasis der Formel (I) mit einer Säure unter den Hydrochloriden, Hydrobromiden, Sulfaten, Citraten, Succinaten, Tartraten, Lactaten und Acetaten ausgewählt sind.

9. Farbmittelzusammensetzungen für Keratinsubstanzen, **dadurch gekennzeichnet, dass** sie in einem zum Färben geeigneten Medium eine wirksame Menge mindestens eines kationischen Nitroanilins auf Monobenzolbasis der Formel (I) nach einem der Ansprüche 1 bis 7 enthält.

10. Zusammensetzung für die Direktfärbung von menschlichen Keratinfasern und insbesondere Haaren, **dadurch gekennzeichnet, dass** sie in einem zum Färben geeigneten Medium eine wirksame Menge mindestens eines kationischen Nitroanilins auf Monobenzolbasis der Formel (I) nach einem der Ansprüche 1 bis 7 enthält.

11. Zusammensetzung nach einem der Ansprüche 9 oder 10, **dadurch gekennzeichnet, dass** sie einen pH-Wert von 3 bis 12 aufweist.

12. Zusammensetzung nach einem der Ansprüche 9 bis 11, **dadurch gekennzeichnet, dass** die kationischen Nitroaniline auf Monobenzolbasis der Formel (I) in einer Konzentration von 0,005 bis 12 Gew.-%, bezogen auf das Gesamtgewicht der Zusammensetzung, enthalten.

13. Zusammensetzung nach Anspruch 12, **dadurch gekennzeichnet, dass** die kationischen Nitroaniline auf Monobenzolbasis der Formel (I) in einer Konzentration von 0,05 bis 6 Gew.-%, bezogen auf das Gesamtgewicht der Zusammensetzung, enthalten.

14. Zusammensetzung nach einem der Ansprüche 9 bis 13, **dadurch gekennzeichnet, dass** das zum Färben geeignete Medium ein wässeriges Medium ist, das aus Wasser und/ oder organischen Lösungsmitteln in Mengenanteilen von 1 bis 40 Gew.-%, bezogen auf das Gesamtgewicht der Zusammensetzung, besteht.

15. Verfahren zum Färben von Keratinfasern, insbesondere menschlichen Keratinfasern, besonders Haaren, durch Direktfärbung, **dadurch gekennzeichnet, dass** eine Farbmittelzusammensetzung nach einem der Ansprüche 9 bis 14 auf die trockenen oder feuchten Keratinfasern aufgetragen wird und die Fasern ohne zwischenzeitliches Spülen getrocknet werden.

16. Verfahren zum Färben von Keratinfasern und insbesondere menschlichen Keratinfasern, besonders Haaren, durch Direktfärbung, **dadurch gekennzeichnet, dass** eine Farbmittelzusammensetzung nach einem der Ansprüche 9 bis 14 auf die trockenen oder feuchten Keratinfasern aufgetragen und, gegebenenfalls nachdem die Zusammensetzung während einer Zeitspanne von 3 bis 60 min auf die Haare einwirken konnte, die Fasern gespült, gegebenenfalls gewaschen, nochmals gespült und dann getrocknet werden.

## Claims

1. Use, as direct dyes in or for the manufacture of dyeing compositions for keratinous substances, in particular for human keratinous fibres and especially the hair, of cationic monobenzene nitroanilines of following formula (I) and their addition salts with an acid: 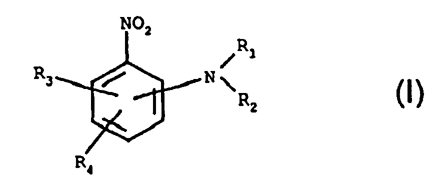
in which formula,
• **R**_{**1**} and **R**_{**2**}, which can be identical or different, represent a hydrogen atom; a Z group defined below; a (C₁-C₆) alkyl radical; a monohydroxy (C₁-C₆) alkyl radical; a polyhydroxy(C₂-C₆)alkyl radical; a (C₁-C₆) alkoxy(C₁-C₆) alkyl radical; an aryl radical; a benzyl radical; a cyano(C₁-C₆)alkyl radical; a carbamyl (C₁-C₆) alkyl radical; an N- (C₁-C₆) alkylcarbamyl (C₁-C₆) alkyl radical; an N, N-di (C₁-C₆) alkylcarbamyl (C₁-C₆) alkyl radical; a thiocarbamyl (C₁-C₆) alkyl radical; a trifluoro (C₁-C₆) alkyl radical; a sulpho (C₁-C₆) alkyl radical; a (C₁-C₆) alkylcarboxy (C₁-C₆) alkyl radical; a (C₁-C₆) alkylsulphinyl (C₁-C₆) alkyl radical; an aminosulphonyl (C₁-C₆) alkyl radical; an N-Z-aminosulphonyl (C₁-C₆) alkyl radical; an N- (C₁-C₆) alkylaminosulphonyl (C₁-C₆) alkyl radical; an N,N-di(C₁-C₆)alkylaminosulphonyl(C₁-C₆) alkyi radical; a (C₁-C₆) alkylcarbonyl (C₁-C₆) alkyl radical; an amino(C₁-C₆)alkyl radical, the alkyl of which is substituted or unsubstituted by one or more hydroxyl radicals; or an amino (C₁-C₆) alkyl radical, the alkyl of which is substituted or unsubstituted by one or more hydroxyl radicals and the amine of which is substituted by one or two identical or different radicals chosen from alkyl, monohydroxy (C₁-C₆) alkyl, polyhydroxy (C₂-C₆) alkyl, (C₁-C₆) alkylcarbonyl, carbamyl, N- (C₁-C₆) alkylcarbamyl or N,N-di(C₁-C₆)alkylcarbamyl, (C₁-C₆) alkylsulphonyl, formyl, trifluoro (C₁-C₆) alkylcarbonyl, (C₁-C₆) alkylcarboxyl or thiocarbamyl radicals, or by a Z group defined below; or being able together to form, with the nitrogen atom to which they are attached, a carbonaceous 5- or 6-membered ring or a 5- or 6-membered ring comprising one or more heteroatoms;
• **R**_{**3**} and **R**_{**4**}**,** which can be identical or different, represent a hydrogen atom; a halogen atom; a Z group defined below; a (C₁-C₆)alkylcarbonyl radical; an amino(C₁-C₆)alkylcarbonyl radical; an N-Z-amino(C₁-C₆)alkylcarbonyl radical; an N-(C₁-C₆) alkylamino (C₁-C₆) alkylcarbonyl radical; an N,N-di(C₁-C₆)alkylamino(C₁-C₆) alkylcarbonyl radical; an amino(C₁-C₆)alkylcarbonyl(C₁-C₆)alkyl radical; an N-Z-amino(C₁-C₆)alkylcarbonyl(C₁-C₆) alkyl radical; an N-(C₁-C₆) alkylamino(C₁-C₆) alkylcarbonyl (C₁-C₆) alkyl radical; an N,N-di(C₁-C₆)alkylamino-(C₁-C₆) alkylcarbonyl (C₁-C₆) alkyl radical; a carboxyl radical; a (C₁-C₆)alkylcarboxyl radical; a (C₁-C₆)alkylsulphonyl radical; an aminosulphonyl radical; an N-Z-aminosulphonyl radical; an N-(C₁-C₆)alkylaminosulphonyl radical; an N,N-di(C₁-C₆)alkylaminosulphonyl radical; an aminosulphonyl(C₁-C₆)alkyl radical; an N-Z-aminosulphonyl(C₁-C₆)alkyl radical; an N-(C₁-C₆) alkylaminosulphonyl (C₁-C₆) alkyl radical; an N,N-di(C₁-C₆)alkylaminosulphonyl(C₁-C₆)alkyl radical; a carbamyl radical; an N-(C₁-C₆)alkylcarbamyl radical; an N,N-di(C₁-C₆)alkylcarbamyl radical; a carbamyl (C₁-C₆) alkyl radical; an N- (C₁-C₆) alkylcarbamyl (C₁-C₆) alkyl radical; an N, N-di (C₁-C₆) alkylcarbamyl (C₁-C₆) alkyl radical; a (C₁-C₆)alkyl radical; a monohydroxy(C₁-C₆)alkyl radical; a polyhydroxy(C₂-C₆)alkyl radical; a (C₁-C₆) alkoxy (C₁-C₆) alkyl radical; a trifluoro (C₁-C₆) - alkyl radical; a cyano radical; an amino (C₁-C₆) alkyl radical, the alkyl of which is substituted or unsubstituted by one or more hydroxyl radicals and the amine of which is substituted or unsubstituted by one or two identical or different radicals chosen from (C₁-C₆) alkyl, monohydroxy (C₁-C₆) alkyl, polyhydroxy (C₂-C₆) alkyl, (C₁-C₆) alkylcarbonyl, carbamyl, N-(C₁-C₆) alkylcarbamyl or N, N-di (C₁-C₆) alkylcarbamyl, (C₁-C₆) alkylsulphonyl, formyl, trifluoro (C₁-C₆) alkylcarbonyl, (C₁-C₆) alkylcarboxyl or thiocarbamyl radicals, or by a Z group defined below; an OR₅ or -SR₅ group defined below; or being able together to form, with the nitrogen atom to which they are attached, a carbonaceous 5- or 6-membered ring or a 5- or 6-membered ring comprising one or more heteroatoms;
• R₅ denotes a hydrogen atom; a (C₁-C₆) alkyl radical; a monohydroxy (C₁-C₆) alkyl radical; a polyhydroxy (C₂-C₆) - alkyl radical; a Z group; a (C₁-C₆) alkoxy (C₁-C₆) alkyl radical; an aryl radical; a benzyl radical; a carboxy (C₁-C₆) alkyl radical; a (C₁-C₆) alkylcarboxy-(C₁-C₆) alkyl radical; a cyano (C₁-C₆) alkyl radical; a carbamyl (C₁-C₆) alkyl radical; an N- (C₁-C₆) alkylcarbamyl (C₁-C₆) alkyl radical; an N, N-di (C₁-C₆) alkylcarbamyl (C₁-C₆) alkyl radical; a trifluoro (C₁-C₆) alkyl radical; an aminosulphonyl (C₁-C₆) alkyl radical; an N-Z-aminosulphonyl (C₁-C₆) alkyl radical; an N- (C₁-C₆) alkylaminosulphonyl (C₁-C₆) alkyl radical; an N,N-di (C₁-C₆) alkylaminosulphonyl (C₁-C₆) alkyl radical; a (C₁-C₆) alkylsulphinyl (C₁-C₆) alkyl radical; a (C₁-C₆)alkylsulphonyl(C₁-C₆)alkyl radical; a (C₁-C₆) alkylcarbonyl (C₁-C₆) alkyl radical; an amino(C₁-C₆)alkyl radical, the alkyl of which is substituted or unsubstituted by one or more hydroxyl radicals; or an amino (C₁-C₆) alkyl radical, the alkyl of which is substituted or unsubstituted by one or more hydroxyl radicals and the amine of which is substituted by one or two identical or different radicals chosen from (C₁-C₆) alkyl, monohydroxy (C₁-C₆) alkyl, polyhydroxy(C₂-C₆) alkyl, (C₁-C₆) alkylcarbonyl, formyl, trifluoro (C₁-C₆) alkylcarbonyl, (C₁-C₆) alkylcarboxyl, carbamyl, N-(C₁-C₆)alkylcarbamyl, N,N-di(C₁-C₆)alkylcarbamyl, thiocarbamyl or (C₁-C₆)alkylsulphonyl radicals, or by a Z group defined below; or being able together to form, with the nitrogen atom to which they are attached, a carbonaceous 5- or 6-membered ring or a 5- or 6-membered ring comprising one or more heteroatoms;
• **Z** is chosen from the unsaturated cationic groups of following formulae (II) and (III) and the saturated cationic groups of following formula (IV): 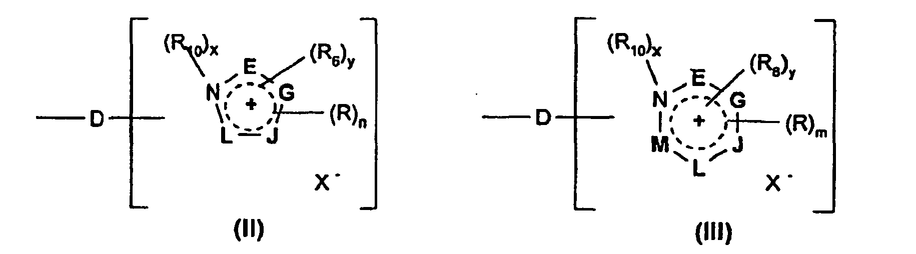 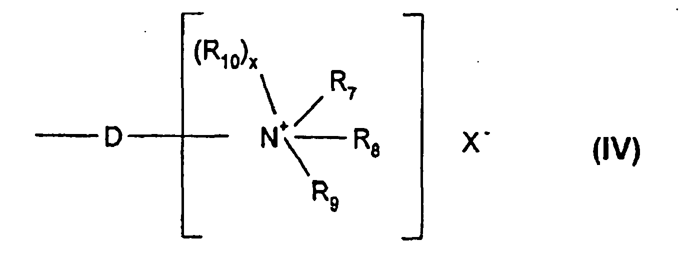
in which:
• D is a linking arm which represents a linear or branched alkyl chain preferably comprising from 1 to 14 carbon atoms which can be interrupted by one or more heteroatoms, such as oxygen, sulphur or nitrogen atoms, and which can be substituted by one or more hydroxyl or (C₁-C₆)alkoxy radicals and which can carry one or more ketone functional groups;
• the E, G, J, L and M vertices, which are identical or different, represent a carbon, oxygen, sulphur or nitrogen atom;
• n is an integer between 0 and 4 inclusive;
• m is an integer between 0 and 5 inclusive;
• the R radicals, which are identical or different, represent a second Z group identical to or different from the first Z group, a halogen atom, a hydroxyl radical, a (C₁-C₆) alkyl radical, a monohydroxy (C₁-C₆) - alkyl radical, a polyhydroxy (C₂-C₆) alkyl radical, a nitro radical, a cyano radical, a cyano (C₁-C₆) alkyl radical, a (C₁-C₆) alkoxy radical, a tri (C₁-C₆) alkylsilyl (C₁-C₆) alkyl radical, an amido radical, a formyl radical, a carboxyl radical, a (C₁-C₆)alkylcarbonyl radical, a thio radical, a thio (C₁-C₆) alkyl radical, a (C₁-C₆) alkylthio radical, an amino radical, an amino radical protected by a (C₁-C₆) alkylcarbonyl, carbamyl or (C₁-C₆) alkylsulphonyl radical, or an NHR" or NR"R" group in which R" and R"', which are identical or different, represent a (C₁-C₆)alkyl radical, a monohydroxy (C₁-C₆) alkyl radical or a polyhydroxy(C₂-C₆)alkyl radical;
• R₆ represents a (C₁-C₆)alkyl radical, a monohydroxy-(C₁-C₆) alkyl radical, a polyhydroxy (C₂-C₆) alkyl radical, a cyano (C₁-C₆) alkyl radical, a tri (C₁-C₆) alkylsilyl (C₁-C₆) alkyl radical, a (C₁-C₆) alkoxy (C₁-C₆) alkyl radical, a carbamyl (C₁-C₆) alkyl radical, a (C₁-C₆)alkylcarboxy(C₁-C₆)alkyl radical, a benzyl radical or a second Z group identical to or different from the first Z group;
• R₇, R₈ and R₉, which are identical or different, represent a (C₁-C₆)alkyl radical, a monohydroxy (C₁-C₆) - alkyl radical, a polyhydroxy(C₂-C₆)alkyl radical, a (C₁-C₆)alkoxy(C₁-C₆) alkyl radical, a cyano(C₁-C₆)alkyl radical, an aryl radical, a benzyl radical, an amido (C₁-C₆) alkyl radical, a tri (C₁-C₆) alkylsilyl-(C₁-C₆) alkyl radical or an amino (C₁-C₆) alkyl radical, the amine of which is protected by a (C₁-C₆)alkylcarbonyl, carbamyl or (C₁-C₆)alkylsulphonyl radical; two of the R₇, R₈ and R₉ radicals can also together form, with the nitrogen atom to which they are attached, a saturated carbonaceous 5- or 6-membered ring or a saturated 5- or 6-membered ring which can comprise one or more heteroatoms, such as, for example, a pyrrolidine ring, a piperidine ring, a piperazine ring or a morpholine ring, it being possible for the said ring to be substituted or unsubstituted by a halogen atom, a hydroxyl radical, a (C₁-C₆) alkyl radical, a monohydroxy(C₁-C₆)alkyl radical, a polyhydroxy(C₂-C₆)alkyl radical, a nitro radical, a cyano radical, a cyano(C₁-C₆)alkyl radical, a (C₁-C₆) alkoxy radical, a tri (C₁-C₆) alkylsilyl (C₁-C₆) alkyl radical, an amido radical, a formyl radical, a carboxyl radical, a keto(C₁-C₆)alkyl radical, a thio radical, a thio(C₁-C₆)alkyl radical, a (C₁-C₆) alkylthio radical, an amino radical or an amino radical protected by a (C₁-C₆)alkylcarbonyl, carbamyl or (C₁-C₆) alkylsulphonyl radical;
one of the R₇, R₈ and R₉ radicals can also represent a second Z group identical to or different from the first Z group;
• R₁₀ represents a (C₁-C₆) alkyl radical; a monohydroxy (C₁-C₆) alkyl radical; a polyhydroxy (C₂-C₆)-alkyl radical; an aryl radical; a benzyl radical; an amino (C₁-C₆) alkyl radical; an amino (C₁-C₆) alkyl radical, the amine of which is protected by a (C₁-C₆) alkylcarbonyl, carbamyl or (C₁-C₆) alkylsulphonyl radical; a carboxy(C₁-C₆)alkyl radical; a cyano (C₁-C₆) alkyl radical; a carbamyl (C₁-C₆) alkyl radical; a trifluoro (C₁-C₆) alkyl radical; a tri (C₁-C₆) alkylsilyl (C₁-C₆) alkyl radical; a sulphonamido (C₁-C₆) alkyl radical; a (C₁-C₆) alkylcarboxy (C₁-C₆) alkyl radical; a (C₁-C₆) alkylsulphinyl (C₁-C₆) alkyl radical; a (C₁-C₆) alkylsulphonyl (C₁-C₆) alkyl radical; a (C₁-C₆) alkylketo(C₁-C₆) alkyl radical; an N- (C₁-C₆) alkylcarbamyl (C₁-C₆) alkyl radical; or an N- (C₁-C₆)alkylsulphonamido (C₁-C₆)alkyl radical;
• x and y are integers equal to 0 or 1; with the following conditions:
- in the unsaturated cationic groups of formula (II):
- when x = 0, the D linking arm is attached to the nitrogen atom,
- when x = 1, the D linking arm is attached to one of the E, G, J or L vertices,
- y can take the value 1 only:
1) when the E, G, J and L vertices simultaneously represent a carbon atom and when the R₆ radical is carried by the nitrogen atom of the unsaturated ring; or else
2) when at least one of the E, G, J and L vertices represents a nitrogen atom to which the R₆ radical is attached;
- in the unsaturated cationic groups of formula (III) :
- when x = 0, the D linking arm is attached to the nitrogen atom,
- when x = 1, the D linking arm is attached to one of the E, G, J, L or M vertices,
- y can take the value 1 only when at least one of the E, G, J, L and M vertices represents a divalent atom and when the R₆ radical is carried by the nitrogen atom of the unsaturated ring;
- in the cationic groups of formula (IV):
- when x = 0, then the D linking arm is attached to the nitrogen atom carrying the R₇ to R₉ radicals,
- when x = 1, then two of the R₇ to R₉ radicals jointly form, with the nitrogen atom to which they are attached, a saturated 5- or 6-membered ring as defined above and the D linking arm is carried by a carbon atom of the said saturated ring;
• **X**⁻ represents a monovalent or divalent anion, **it being understood:**
- that the number of Z unsaturated cationic groups of formula (II) in which at least one of the E, G, J and L vertices represents a nitrogen atom is at least equal to 1, and
- that, when one and only one of the R₁ or R₂ or R₅ radicals denotes a Z group in which the D linking arm represents an alkyl chain comprising a ketone functional group, then the said ketone functional group is not directly attached to the nitrogen atom of the NR₁R₂ group or to the oxygen atom of the OR₅ group.

2. Use according to Claim 1, **characterized in that** the rings of the Z unsaturated groups of formula (II) are chosen from the pyrrole, imidazole, pyrazole, oxazole, thiazole and triazole rings.

3. Use according to Claim 1, **characterized in that** the rings of the Z unsaturated groups of formula (III) are chosen from the pyridine, pyrimidine, pyrazine, oxazine and triazine rings.

4. Use according to any one of the preceding claims, **characterized in that**, in the formula (IV), two of the R₈, R₉ and R₁₀ radicals form a pyrrolidine ring, a piperidine ring, a piperazine ring or a morpholine ring, it being possible for the said ring to be substituted or unsubstituted by a halogen atom, a hydroxyl radical, a (C₁-C₆) alkyl radical, a monohydroxy (C₁-C₆) alkyl radical, a polyhydroxy (C₂-C₆) - alkyl radical, a nitro radical, a cyano radical, a cyano (C₁-C₆) alkyl radical, a (C₁-C₆) alkoxy radical, a tri(C₁-C₆)alkylsilyl(C₁-C₆)alkyl radical, an amido radical, a formyl radical, a carboxyl radical, a (C₁-C₆)alkylcarbonyl radical, a thio radical, a thio (C₁-C₆) alkyl radical, a (C₁-C₆) alkylthio radical, an amino radical or an amino radical protected by a (C₁-C₆) alkylcarbonyl, carbamyl or (C₁-C₆) alkylsulphonyl radical.

5. Use according to any one of the preceding claims, **characterized in that** X⁻ is chosen from a halogen atom, a hydroxide, a hydrogen sulphate or a (C₁-C₆) alkyl sulphate.

6. Use according to any one of the preceding claims, **characterized in that** the cationic monobenzene nitroanilines are chosen from the following compounds:
- 3-[3-(4,5-dichloro-2-nitrophenylamino)propyl]-1-methyl-3H-imidazol-1-ium methyl sulphate,
- 3-[3-(4-chloro-5-methoxy-2-nitrophenylamino)propyl]-1-methyl-3H-imidazol-1-ium methyl sulphate,
- 3-[3-(4-chloro-5-methylsulphanyl-2-nitrophenylamino)-propyl]-1-methyl-3H-imidazol-1-ium methyl sulphate,
- 1,3-dimethyl-2-(3-methylamino-4-nitrophenyl-sulphanyl)-3H-imidazol-1-ium methyl sulphate,
- 1-{2-[5-(2-hydroxyethylamino)-2-methyl-4-nitrophenoxy]ethyl}-3-methyl-3H-imidazol-1-ium bromide,
- 3-[3-(4-methoxy-2-nitrophenylamino)propyl]-1-methyl-3H-imidazol-1-ium methyl sulphate hydrate,
- 3-(3-[4-(2-hydroxyethoxy)-2-nitrophenylamino]propyl)-1-methyl-3H-imidazol-1-ium methyl sulphate,
- 3-methyl-1-[2-(4-nitrophenylamino)ethyl]-3H-imidazol-1-ium bromide,
- 1-methyl-3-[3-(4-nitrophenylamino)propyl]-3H-imidazol-1-ium methyl sulphate,
- 1-[3-(2-amino-5-nitrophenoxy)propyl]-3-methyl-3H-imidazol-1-ium chloride hydrochloride,
- 3-[3-(4,5-dichloro-2-nitrophenylamino)propyl]-1-[3-(trimethylsilanyl)propyl]-3H-imidazol-1-ium chloride hydrate,
- 1-[3-(2-amino-5-nitrophenoxy)propyl]-2-methyl-2H-pyrazol-1-ium bromide,
- 1-methyl-3-[3-(2-nitrophenylamino)propyl]-3H-imidazol-1-ium methyl sulphate,
- 1-(3-chloropropyl)-3-[3-(4-nitrophenylamino)propyl]-3H-imidazol-1-ium chloride,
- 1-(2-hydroxyethyl)-3-[3-(4-nitrophenylamino)propyl]-3H-imidazol-1-ium chloride,
- 3-[3-(4-benzyloxy-2-nitrophenylamino)propyl]-1-methyl-3H-imidazol-1-ium methyl sulphate,
- 3-[3-(2-cyano-4-nitrophenylamino)propyl]-1-methyl-3H-imidazol-1-ium methyl sulphate,
- 1-methyl-3-[3-(2-methyl-4-nitrophenylamino)propyl]-3H-imidazol-1-ium methyl sulphate,
- 3-[3-(2-fluoro-4-nitrophenylamino)propyl]-1-methyl-3H-imidazol-1-ium methyl sulphate,
- 1-[2-(2-methoxy-4-nitrophenylamino)ethyl]-3-methyl-3H-imidazol-1-ium bromide,
- 3-[3-(3-hydroxy-4-nitrophenylamino)propyl]-1-methyl-3H-imidazol-1-ium methyl sulphate,
- 3-[3-(2-chloro-4-nitrophenylamino)propyl]-1-methyl-3H-imidazol-1-ium methyl sulphate,
- 2-methyl-1-[2-(4-nitrophenylamino)ethyl]-2H-pyrazol-1-ium bromide,
- 1-(3-bromo-2-hydroxypropyl)-3-[3-(4-nitrophenyl-amino)propyl]-3H-imidazol-1-ium bromide,
- 1-(3-trimethylammonio-2-hydroxypropyl)-3-[3-(4-nitrophenylamino)propyl]-3H-imidazol-1-ium dichloride,
- diethyl(2-hydroxyethyl)[4-(4-nitrophenylamino)-pentyl]ammonium chloride,
- 3-[3-(4-chloro-2-nitrophenylamino)propyl]-1-methyl-3H-imidazol-1-ium methyl sulphate,
- 3-[3-(2-chloro-6-nitrophenylamino)propyl]-1-methyl-3H-imidazol-1-ium methyl sulphate,
- 1-methyl-3-[2-(4-nitrophenylamino)butyl]-3H-imidazol-1-ium chloride,
- 3-methyl-1-[2-methyl-2-(4-nitrophenylamino)propyl]-3H-imidazol-1-ium chloride,
- 1-{2-[(2-(3-methyl-3H-imidazol-1-io)ethyl](4-nitrophenyl)amino]ethyl}-3-methyl-3H-imidazol-1-ium dichloride.

7. Use according to Claim 6, **characterized in that** the cationic monobenzene nitroanilines are of following formulae (I)₁ to (I)₁₁:

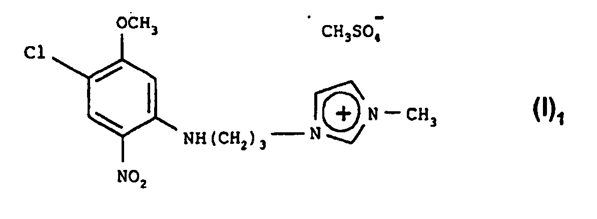

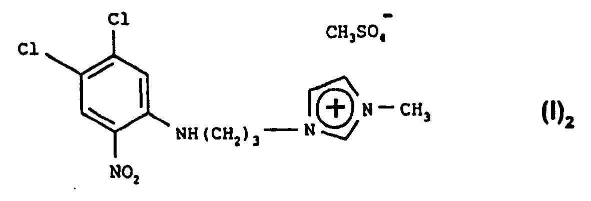

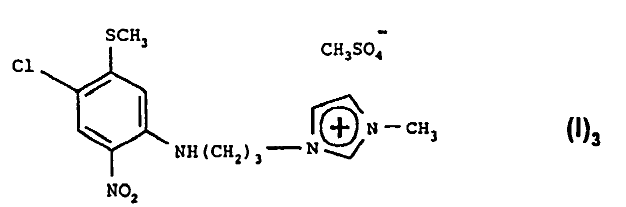

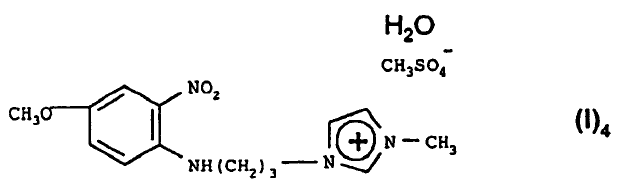

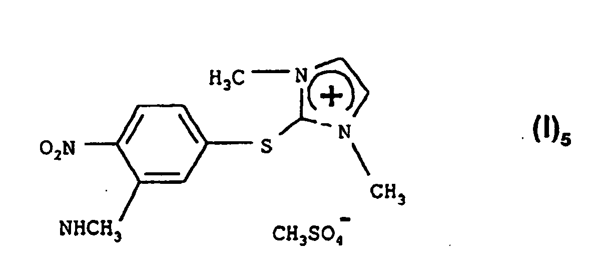

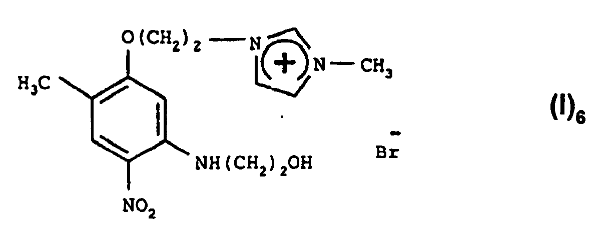

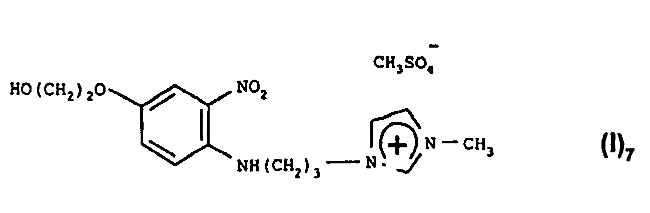

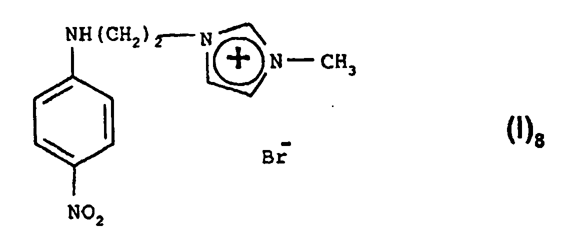

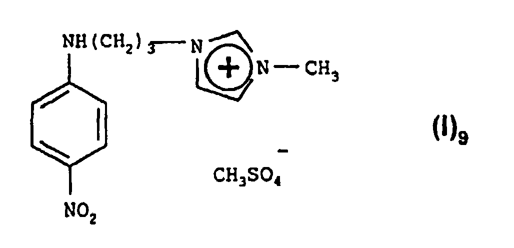

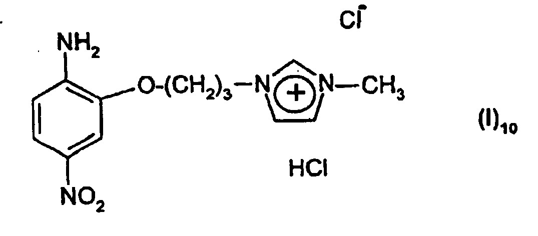

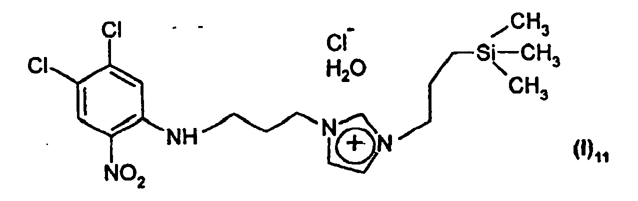

8. Use according to any one of the preceding claims, **characterized in that** the addition salts with an acid of the cationic monobenzene nitroanilines of formula (I) are chosen from hydrochlorides, hydrobromides, sulphates, citrates, succinates, tartrates, lactates and acetates.

9. Dyeing composition for keratinous substances, **characterized in that** it comprises, in a medium appropriate for dyeing, an effective amount of at least one cationic monobenzene nitroaniline of formula (I) defined in any one of Claims 1 to 7.

10. Direct dyeing composition for human keratinous fibres and in particular the hair, **characterized in that** it comprises, in a medium appropriate for dyeing, an effective amount of at least one cationic monobenzene nitroaniline of formula (I) defined in any one of Claims 1 to 7.

11. Composition according to Claims 9 and 10, **characterized in that** it has a pH of between 3 and 12.

12. Composition according to any one of Claims 9 to 11, **characterized in that** the cationic monobenzene nitroanilines of formula (I) are present in a concentration ranging from 0.005 to 12% by weight with respect to the total weight of the composition.

13. Composition according to Claim 12, **characterized in that** the cationic monobenzene nitroanilines of formula (I) are present in a concentration ranging from 0.05 to 6% by weight with respect to the total weight of the composition.

14. Composition according to any one of Claims 9 to 13, **characterized in that** the medium appropriate for dyeing is an aqueous medium composed of water and/or of organic solvents in proportions of between 1 and 40% by weight with respect to the total weight of the composition.

15. Process for dyeing keratinous fibres and in particular human keratinous fibres, especially the hair, by direct colouring, **characterized in that** a dyeing composition as defined in any one of Claims 9 to 14 is applied to dry or wet keratinous fibres and **in that** these fibres are dried without intermediate rinsing.

16. Process for dyeing keratinous fibres and in particular human keratinous fibres, especially the hair, by direct colouring, **characterized in that** a dyeing composition as defined in any one of Claims 9 to 14 is applied to dry or wet keratinous fibres and **in that**, after having optionally allowed the composition to act on the fibres for an exposure time ranging from 3 to 60 minutes, the fibres are rinsed, are optionally washed, are again rinsed and are then dried.
